(19) Europäisches Patentamt
European Patent Office
Office européen des brevets

(11) **EP 4 717 339 A2**

(12) **EUROPEAN PATENT APPLICATION**

(43) Date of publication:
**01.04.2026 Bulletin 2026/14**

(21) Application number: **25207442.2**

(22) Date of filing: **24.07.2019**

(51) International Patent Classification (IPC):
***B01D 15/38*** *(2006.01)*

(52) Cooperative Patent Classification (CPC):
**B01D 15/166; B01D 15/362; B01D 15/363;
B01D 15/3847; C07K 1/22; C07K 16/065**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.07.2018 US 201862703097 P**

(62) Document number(s) of the earlier application(s) in
accordance with Art. 76 EPC:
**19841547.3 / 3 826 743**

(71) Applicant: **Merck Sharp & Dohme LLC
Rahway, New Jersey 07065-0907 (US)**

(72) Inventors:
• **CHMIELOWSKI, Rebecca, A
Kenilworth, New Jersey 07033 (US)**
• **INSAIDOO, Francis, K
Kenilworth, New Jersey 07033 (US)**
• **MILLER, Justin, B
Kenilworth, New Jersey 07033 (US)**

• **ROUSH, David, J
Kenilworth, New Jersey 07033 (US)**
• **SHAH, Darshini
Kenilworth, New Jersey 08873 (US)**
• **WELSH, John, P.
Kenilworth, New Jersey 07033 (US)**

(74) Representative: **Merck Sharp & Dohme LLC
120 Moorgate
London EC2M 6UR (GB)**

Remarks:
•This application was filed on 08-10-2025 as a
divisional application to the application mentioned
under INID code 62.
•Claims filed after the date of filing of the application /
after the date of receipt of the divisional application
(Rule 68(4) EPC).

(54) **METHODS OF SEPARATING HOST CELL LIPASES FROM A PRODUCTION PROTEIN IN CHROMATOGRAPHIC PROCESSES**

(57)    Provided herein are methods of separating host cell lipases from a production protein in chromatographic processes and methods of improving polysorbate-80 stability in a production protein formulation by separating host cell lipases from the production protein using chromatographic processes. Also provided are pharmaceutical compositions comprising less than 1 ppm of a host cell lipase.

FIG.12

**Description**

**CROSS REFERENCE TO RELATED APPLICATIONS**

This application claims the benefit of priority to U.S. Provisional Application No. 62/703,097, filed on July 25, 2018, the disclosure of which is incorporated herein by its entirety.

**I. FIELD**

Provided herein are methods of separating host cell proteins (HCP) (*e.g.*, lipases) from a production protein (*e.g.*, monoclonal antibody) in chromatographic processes. Also provided herein are methods of improving polysorbate-80 (PS-80) stability in a production protein formulation (*e.g.*, drug substance formulation or drug product formulation) by separating HCP (*e.g.*, lipases) from the production protein (*e.g.*, monoclonal antibody) using chromatographic processes.

**II. BACKGROUND OF THE INVENTION**

In bioprocessing and manufacturing of production proteins (*e.g.*, monoclonal antibodies), HCP (*e.g.*, lipases) constitute part of the impurities that are often difficult to remove from the production proteins. Such impurities can cause various issues in the safety and efficacy of biopharmaceuticals. Regulatory agencies throughout the world require that biopharmaceutical products meet certain acceptance criteria, including the level of impurities and tests for detection and quantification of impurities. Thus, it is desirable to develop efficient and effective processes to remove HCP (*e.g.*, lipases) from production proteins (*e.g.*, monoclonal antibodies).

**III. SUMMARY**

The present disclosure provides methods of separating HCP (*e.g.*, lipases) from a production protein (*e.g.*, monoclonal antibody) in chromatographic processes as well as methods of improving PS-80 stability in a production protein formulation (*e.g.*, drug substance formulation or drug product formulation) by separating HCP (*e.g.*, lipases) from a production protein (*e.g.*, monoclonal antibody) using chromatographic processes. The disclosure is based, at least in part, on the discovery that the HCP (*e.g.*, lipases) and the production protein (*e.g.*, monoclonal antibody) can be sufficiently separated under operating conditions where the separation factor ($\alpha$) between the two proteins and/or the partition coefficient ($K_p$) for the HCP (*e.g.*, lipase) reach certain ranges of numeric values.

In one aspect, provided herein is a method of separating a host cell lipase from a production protein through a chromatographic process, comprising:

(a) passing a load fluid comprising the lipase and the production protein through a chromatographic resin under a loading operating condition; and
(b) collecting the production protein in a flowthrough;

wherein separation factor ($\alpha$) is the ratio of the partition coefficient ($K_p$) for the lipase to the $K_p$ for the production protein, and wherein log $\alpha$ is larger than 0.5 under the loading operating condition.

In certain embodiments, log $\alpha$ is larger than 1.0 under the loading operating condition.

In some embodiments, the log $K_p$ for the lipase is larger than 1.0 under the loading operating condition. In other embodiments, the log $K_p$ for the lipase is larger than 1.5 under the loading operating condition.

In certain embodiments, log $\alpha$ is larger than 0.5 and the log $K_p$ for the lipase is larger than 1.0 under the loading operating condition. In some embodiments, log $\alpha$ is larger than 0.5 and the log $K_p$ for the lipase is larger than 1.5 under the loading operating condition. In other embodiments, log $\alpha$ is larger than 1.0 and the log $K_p$ for the lipase is larger than 1.0 under the loading operating condition. In yet other embodiments, log $\alpha$ is larger than 1.0 and the log $K_p$ for the lipase is larger than 1.5 under the loading operating condition.

In another aspect, provided herein is a method of separating a host cell lipase from a production protein through a chromatographic process, comprising:

(a) passing a load fluid comprising the lipase and the production protein through a chromatographic resin; and
(b) eluting the production protein from the chromatographic resin with an elution solution under an elution operating condition;

wherein $\alpha$ is the ratio of $K_p$ for the lipase to the $K_p$ for the production protein, and wherein log $\alpha$ is larger than 0.5 under the elution operating condition.

In certain embodiments, log $\alpha$ is larger than 1.0 under the elution operating condition.

In some embodiments, the log $K_p$ for the lipase is larger than 1.0 under the elution operating condition. In other embodiments, the log $K_p$ for the lipase is larger than 1.5 under the elution operating condition.

In certain embodiments, log $\alpha$ is larger than 0.5 and the log $K_p$ for the lipase is larger than 1.0 under the elution operating condition. In some embodiments, log $\alpha$ is larger than 0.5 and the log $K_p$ for the lipase is larger than 1.5 under the elution operating condition. In other embodiments, log $\alpha$ is larger than 1.0 and the log $K_p$ for the lipase is larger than 1.0 under the elution operating condition. In yet other embodiments, log $\alpha$ is larger than 1.0 and the log $K_p$ for the lipase is larger than 1.5 under the elution operating condition.

In some embodiments of various methods provided herein, the lipase is a Chinese Hamster Ovary (CHO) cell lipase.

In certain embodiments, the lipase is selected from the group consisting of phospholipase B-like 2 (PLBL2), lipoprotein lipase (LPL), lysosomal phospholipase A2 (LPLA2), phospholipase A2 VII (LP-PLA2), and lysosomal acid lipase A (LAL). In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2, LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2, LP-PLA2, and LAL.

In certain embodiments, the CHO cell lipase is selected from the group consisting of PLBL2, LPL, LPLA2, LP-PLA2, and LAL. In one embodiment, the CHO cell lipase is PLBL2. In another embodiment, the CHO cell lipase is LPL. In yet another embodiment, the CHO cell lipase is LPLA2. In one embodiment, the CHO cell lipase is LP-PLA2. In another embodiment, the CHO cell lipase is LAL. In still another embodiment, the CHO cell lipase includes two, three, four, five, six, seven, eight, nine, ten, or more different CHO cell lipases. In yet still another embodiment, the CHO cell lipase includes two, three, four, or five different CHO cell lipases selected from the group consisting of PLBL2, LPL, LPLA2, LP-PLA2, and LAL. In one embodiment, the CHO cell lipase includes PLBL2 and LPL. In another embodiment, the CHO cell lipase includes PLBL2 and LPLA2. In yet another embodiment, the CHO cell lipase includes PLBL2 and LP-PLA2. In still another embodiment, the CHO cell lipase includes PLBL2 and LAL. In one embodiment, the CHO cell lipase includes LPL and LPLA2. In another embodiment, the CHO cell lipase includes LPL and LP-PLA2. In yet another embodiment, the CHO cell lipase includes LPL and LAL. In still another embodiment, the CHO cell lipase includes LPLA2 and LP-PLA2. In one embodiment, the CHO cell lipase includes LPLA2 and LAL. In another embodiment, the CHO cell lipase includes LP-PLA2 and LAL. In yet another embodiment, the CHO cell lipase includes PLBL2, LPL, and LPLA2. In still another embodiment, the CHO cell lipase includes PLBL2, LPL, and LP-PLA2. In one embodiment, the CHO cell lipase includes PLBL2, LPL, and LAL. In another embodiment, the CHO cell lipase includes PLBL2, LPLA2, and LP-PLA2. In yet another embodiment, the CHO cell lipase includes PLBL2, LPLA2, and LAL. In still another embodiment, the CHO cell lipase includes PLBL2, LP-PLA2, and LAL. In one embodiment, the CHO cell lipase includes LPL, LPLA2, and LP-PLA2. In another embodiment, the CHO cell lipase includes LPL, LPLA2, and LAL. In yet another embodiment, the CHO cell lipase includes LPL, LP-PLA2, and LAL. In still another embodiment, the CHO cell lipase includes LPLA2, LP-PLA2, and LAL. In one embodiment, the CHO cell lipase includes PLBL2, LPL, LPLA2, and LP-PLA2. In another embodiment, the CHO cell lipase includes PLBL2, LPL, LPLA2, and LAL. In yet another embodiment, the CHO cell lipase includes PLBL2, LPL, LP-PLA2, and LAL. In still another embodiment, the CHO cell lipase includes PLBL2, LPLA2, LP-PLA2, and LAL. In yet still another embodiment, the CHO cell lipase includes PLBL2, LPL, LPLA2, LP-PLA2, and LAL.

In some embodiments of various methods provided herein, the chromatographic resin is an ion exchange (IEX) resin. In other embodiments, the chromatographic resin is a hydrophobic interaction (HIC) resin. In one embodiment, the IEX resin is a cation exchange (CEX) resin. In another embodiment, the CEX resin is a mixed mode CEX resin. In yet another embodiment, the IEX resin is an anion exchange (AEX) resin. In still another embodiment, the AEX resin is a mixed mode AEX resin.

In certain embodiments of various methods using a CEX resin or a mixed mode CEX resin, the pH of the operating condition is below about 6.0. In some embodiments of various methods using a CEX resin or a mixed mode CEX resin, the pH of the operating condition is below about 5.5. In other embodiments of various methods using a CEX resin or a mixed mode CEX resin, the pH of the operating condition is below about 5.0. In yet other embodiments of various methods using a CEX resin or a mixed mode CEX resin, the pH of the operating condition is from about 4.5 to about 5.5. In still other embodiments of various methods using a CEX resin or a mixed mode CEX resin, the pH of the operating condition is from about 4.5 to about 5.0. In certain embodiments of various methods using a CEX resin or a mixed mode CEX resin, the pH of the operating condition is from about 5.0 to about 5.5. In some embodiments of various methods using a CEX resin or a mixed mode CEX resin, the pH of the operating condition is from about 4.9 to about 5.3.

In certain embodiments of various methods using an AEX resin or a mixed mode AEX resin, the pH of the operating condition is above about 6.5. In some embodiments of various methods using an AEX resin or a mixed mode AEX resin, the pH of the operating condition is above about 6.9. In other embodiments of various methods using an AEX resin or a mixed mode AEX resin, the pH of the operating condition is above about 7.2. In yet other embodiments of various methods using an AEX resin or a mixed mode AEX resin, the pH of the operating condition is from about 6.9 to about 7.9. In still other embodiments of various methods using an AEX resin or a mixed mode AEX resin, the pH of the operating condition is from about 7.2 to about 7.5. In certain embodiments of various methods using an AEX resin or a mixed mode AEX resin, the pH of the operating condition is from about 7.5 to about 7.8.

In certain embodiments of various methods provided herein, the operating condition further comprises modulating the ionic strength and/or conductivity of the operating solution by adding a salt. In one embodiment, the operating condition further comprises modulating the ionic strength of the operating solution by adding a salt. In another embodiment, the operating condition further comprises modulating the conductivity of the operating solution by adding a salt. In yet another embodiment, the operating condition further comprises modulating the ionic strength and conductivity of the operating solution by adding a salt. In some embodiments, the effect of adding a salt is to achieve the desired log $\alpha$. In other embodiments, the effect of adding a salt is to achieve the desired log $K_p$ for the lipase. In yet other embodiments, the effect of adding a salt is to achieve the desired log $\alpha$ and the desired log $K_p$ for the lipase.

In some embodiments, the salt in the operating solution is selected from the group consisting of sodium chloride, sodium acetate, sodium phosphate, ammonium sulfate, sodium sulfate, and Tris-HCl. In one embodiment, the salt is sodium chloride. In another embodiment, the salt is sodium acetate. In yet another embodiment, the salt is sodium phosphate. In still another embodiment, the salt is ammonium sulfate. In one embodiment, the salt is sodium sulfate. In another embodiment, the salt is Tris-HCl.

In a specific embodiment, the concentration of sodium chloride in the operating solution is from about 100 mM to about 225 mM, the chromatographic resin is CEX, and the pH of the operating condition is from about 5.0 to about 6.0.

In another specific embodiment, the concentration of sodium chloride in the operating solution is from about 150 mM to about 180 mM, the chromatographic resin is CEX, and the pH of the operating condition is from about 5.0 to about 6.0.

In yet another specific embodiment, the concentration of sodium acetate in the operating solution is from about 100 mM to about 200 mM, the chromatographic resin is AEX; the pH of the operating condition is from about 6.9 to about 7.8.

In still another specific embodiment, the concentration of sodium sulfate in the operating solution is from about 500 mM to about 620 mM, the chromatographic resin is HIC, and the pH of the operating condition is about 7.

In yet still another specific embodiment, the concentration of sodium sulfate in the operating solution is from about 510 mM to about 560 mM, the chromatographic resin is HIC, and the pH of the operating condition is about 7.

In yet another aspect, provided herein is a method of separating PLBL2 from a production protein through a mixed mode AEX chromatographic process, comprising:

(a) passing a load fluid comprising PLBL2 and the production protein through a mixed mode AEX resin; and
(b) collecting the production protein in a flowthrough;

wherein the pH of the load fluid is from about pH 7.2 to about pH 7.6, and wherein the load fluid does not comprise a salt.

In still another aspect, provided herein is a method of separating PLBL2 from a production protein through a CEX chromatographic process, comprising:

(a) passing a load fluid comprising PLBL2 and the production protein through a CEX resin; and
(b) eluting the production protein from the CEX resin with an elution solution;

wherein the pH of the elution solution is from about pH 4.9 to about pH 5.3, and wherein the elution solution further comprises from about 120 mM to about 175 mM sodium chloride.

In one embodiment, the method of separating PLBL2 from a production protein through a CEX chromatographic process comprises:

(a) passing a load fluid comprising PLBL2 and the production protein through a CEX resin; and
(b) eluting the production protein from the CEX resin with an elution solution;

wherein the pH of the elution solution is about pH 5.1, and wherein the elution solution further comprises about 150 mM sodium chloride.

In yet another embodiment, the method of separating PLBL2 from a production protein through a CEX chromatographic process comprises:

(a) passing a load fluid comprising PLBL2 and the production protein through a CEX resin; and
(b) eluting the production protein from the CEX resin with an elution solution;

wherein the pH of the elution solution is about pH 5.1, and wherein the elution solution further comprises about 165 mM sodium chloride.

In still another aspect, provided herein is a method of separating LPLA2 from a production protein through a CEX chromatographic process, comprising:

(a) passing a load fluid comprising LPLA2 and the production protein through a CEX resin; and
(b) eluting the production protein from the CEX resin with an elution solution;

wherein the pH of the elution solution is from about pH 5.0 to about pH 5.4, and wherein the elution solution further comprises from about 150 mM to about 275 mM sodium chloride.

In one embodiment, the method of separating LPLA2 from a production protein through a CEX chromatographic process comprises:

(a) passing a load fluid comprising LPLA2 and the production protein through a CEX resin; and
(b) eluting the production protein from the CEX resin with an elution solution;

wherein the pH of the elution solution is about pH 5.1, and wherein the elution solution further comprises about 150 mM sodium chloride.

In another embodiment, the method of separating LPLA2 from a production protein through a CEX chromatographic process comprises:

(a) passing a load fluid comprising LPLA2 and the production protein through a CEX resin; and
(b) eluting the production protein from the CEX resin with an elution solution;

wherein the pH of the elution solution is about pH 5.1, and wherein the elution solution further comprises about 200 mM sodium chloride.

In yet another embodiment, the method of separating LPLA2 from a production protein through a CEX chromatographic process comprises:

(a) passing a load fluid comprising LPLA2 and the production protein through a CEX resin; and
(b) eluting the production protein from the CEX resin with an elution solution;

wherein the pH of the elution solution is about pH 5.1, and wherein the elution solution further comprises about 250 mM sodium chloride.

In more embodiments of the various methods provided herein, the load fluid is an eluate from a prior chromatographic process. In one embodiment, the prior chromatographic process comprises an affinity chromatography. In another embodiment, the prior chromatographic process comprises an affinity chromatography followed by a non-affinity chromatography. In yet another embodiment, the affinity chromatography is a protein A chromatography. In still another embodiment, the non-affinity chromatography is an AEX chromatography. In yet still another embodiment, the prior chromatographic process comprises a protein A chromatography followed by an AEX chromatography.

In yet still another aspect, provided herein is a method of improving PS-80 stability in a production protein formulation, comprising:

(a) passing a load fluid comprising a host cell lipase and the production protein through a chromatographic resin under a loading operating condition;

(b) collecting the production protein in a flowthrough; and

(c) formulating the production protein so that the production protein formulation is a PS-80-containing formulation;

wherein separation factor ($\alpha$) is the ratio of the partition coefficient ($K_p$) for the lipase to the $K_p$ for the production protein, and wherein log $\alpha$ is larger than 0.5 under the loading operating condition.

In certain embodiments, log $\alpha$ is larger than 1.0 under the loading operating condition.

In some embodiments, the log $K_p$ for the lipase is larger than 1.0 under the loading operating condition. In other embodiments, the log $K_p$ for the lipase is larger than 1.5 under the loading operating condition.

In certain embodiments, log $\alpha$ is larger than 0.5 and the log $K_p$ for the lipase is larger than 1.0 under the loading operating condition. In some embodiments, log $\alpha$ is larger than 0.5 and the log $K_p$ for the lipase is larger than 1.5 under the loading operating condition. In other embodiments, log $\alpha$ is larger than 1.0 and the log $K_p$ for the lipase is larger than 1.0 under the loading operating condition. In yet other embodiments, log $\alpha$ is larger than 1.0 and the log $K_p$ for the lipase is larger than 1.5 under the loading operating condition.

In another aspect, provided herein is a method of improving PS-80 stability in a production protein formulation, comprising:

(a) passing a load fluid comprising a host cell lipase and the production protein through a chromatographic resin;

(b) eluting the production protein from the chromatographic resin with an elution solution under an elution operating condition; and

(c) formulating the production protein so that the production protein formulation is a PS-80-containing solution;

wherein $\alpha$ is the ratio of $K_p$ for the lipase to the $K_p$ for the production protein, and wherein log $\alpha$ is larger than 0.5 under the elution operating condition.

In certain embodiments, log $\alpha$ is larger than 1.0 under the elution operating condition.

In some embodiments, the log $K_p$ for the lipase is larger than 1.0 under the elution operating condition. In other embodiments, the log $K_p$ for the lipase is larger than 1.5 under the elution operating condition.

In certain embodiments, log $\alpha$ is larger than 0.5 and the log $K_p$ for the lipase is larger than 1.0 under the elution operating condition. In some embodiments, log $\alpha$ is larger than 0.5 and the log $K_p$ for the lipase is larger than 1.5 under the elution operating condition. In other embodiments, log $\alpha$ is larger than 1.0 and the log $K_p$ for the lipase is larger than 1.0 under the elution operating condition. In yet other embodiments, log $\alpha$ is larger than 1.0 and the log $K_p$ for the lipase is larger than 1.5 under the elution operating condition.

In some embodiments of various methods provided herein, the lipase is a Chinese Hamster Ovary (CHO) cell lipase.

In certain embodiments, the lipase is selected from the group consisting of PLBL2, LPL, LPLA2, LP-PLA2, and LAL. In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2 , LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2 , LP-PLA2, and LAL.

In certain embodiments, the CHO cell lipase is selected from the group consisting of PLBL2, LPL, LPLA2, LP-PLA2, and LAL. In one embodiment, the CHO cell lipase is PLBL2. In another embodiment, the CHO cell lipase is LPL. In yet another embodiment, the CHO cell lipase is LPLA2. In one embodiment, the CHO cell lipase is LP-PLA2. In another embodiment, the CHO cell lipase is LAL. In still another embodiment, the CHO cell lipase includes two, three, four, five, six, seven, eight, nine, ten, or more different CHO cell lipases. In yet still another embodiment, the CHO cell lipase includes two, three, four, or five different CHO cell lipases selected from the group consisting of PLBL2, LPL, LPLA2 , LP-PLA2, and LAL. In one embodiment, the CHO cell lipase includes PLBL2 and LPL. In another embodiment, the CHO cell lipase includes PLBL2 and LPLA2. In yet another embodiment, the CHO cell lipase includes PLBL2 and LP-PLA2. In still another embodiment, the CHO cell lipase includes PLBL2 and LAL. In one embodiment, the CHO cell lipase includes LPL and LPLA2. In another embodiment, the CHO cell lipase includes LPL and LP-PLA2. In yet another embodiment, the CHO cell lipase includes LPL and LAL. In still another embodiment, the CHO cell lipase includes LPLA2 and LP-PLA2. In one embodiment, the CHO cell lipase includes LPLA2 and LAL. In another embodiment, the CHO cell lipase includes LP-PLA2 and LAL. In yet another embodiment, the CHO cell lipase includes PLBL2, LPL, and LPLA2. In still another embodiment, the CHO cell lipase includes PLBL2, LPL, and LP-PLA2. In one embodiment, the CHO cell lipase includes PLBL2, LPL, and LAL. In another embodiment, the CHO cell lipase includes PLBL2, LPLA2, and LP-PLA2. In yet another embodiment, the CHO cell lipase includes PLBL2, LPLA2, and LAL. In still another embodiment, the CHO cell lipase includes PLBL2, LP-PLA2, and LAL. In one embodiment, the CHO cell lipase includes LPL, LPLA2, and LP-PLA2. In another embodiment, the CHO cell lipase includes LPL, LPLA2, and LAL. In yet another embodiment, the CHO cell lipase includes LPL, LP-PLA2, and LAL. In still another embodiment, the CHO cell lipase includes LPLA2, LP-PLA2, and LAL. In one embodiment, the CHO cell lipase includes PLBL2, LPL, LPLA2, and LP-PLA2. In another embodiment, the CHO cell lipase includes PLBL2, LPL, LPLA2, and LAL. In yet another embodiment, the CHO cell lipase includes PLBL2, LPL, LP-PLA2, and LAL. In still another embodiment, the CHO cell lipase includes PLBL2, LPLA2, LP-PLA2, and LAL. In yet still another embodiment, the CHO cell lipase includes PLBL2, LPL, LPLA2 , LP-PLA2, and LAL.

In some embodiments of various methods provided herein, the chromatographic resin is an IEX resin. In other embodiments, the chromatographic resin is a HIC resin. In one embodiment, the IEX resin is a CEX resin. In another embodiment, the CEX resin is a mixed mode CEX resin. In yet another embodiment, the IEX resin is an AEX resin. In still another embodiment, the AEX resin is a mixed mode AEX resin.

In certain embodiments of various methods using a CEX resin or a mixed mode CEX resin, the pH of the operating condition is below about 6.0. In some embodiments of various methods using a CEX resin or a mixed mode CEX resin, the pH of the operating condition is below about 5.5. In other embodiments of various methods using a CEX resin or a mixed mode CEX resin, the pH of the operating condition is below about 5.0. In yet other embodiments of various methods using a CEX resin or a mixed mode CEX resin, the pH of the operating condition is from about 4.5 to about 5.5. In still other embodiments of various methods using a CEX resin or a mixed mode CEX resin, the pH of the operating condition is from about 4.5 to about 5.0. In certain embodiments of various methods using a CEX resin or a mixed mode CEX resin, the pH of the operating condition is from about 5.0 to about 5.5. In some embodiments of various methods using a CEX resin or a mixed mode CEX resin, the pH of the operating condition is from about 4.9 to about 5.3.

In certain embodiments of various methods using an AEX resin or a mixed mode AEX resin, the pH of the operating condition is above about 6.5. In some embodiments of various methods using an AEX resin or a mixed mode AEX resin, the pH of the operating condition is above about 6.9. In other embodiments of various methods using an AEX resin or a mixed mode AEX resin, the pH of the operating condition is above about 7.2. In yet other embodiments of various methods using an AEX resin or a mixed mode AEX resin, the pH of the operating condition is from about 6.9 to about 7.9. In still other embodiments of various methods using an AEX resin or a mixed mode AEX resin, the pH of the operating condition is from about 7.2 to about 7.5. In certain embodiments of various methods using an AEX resin or a mixed mode AEX resin, the pH of the operating condition is from about 7.5 to about 7.8.

In certain embodiments of various methods provided herein, the operating condition further comprises modulating the ionic strength and/or conductivity of the operating solution by adding a salt. In one embodiment, the operating condition further comprises modulating the ionic strength of the operating solution by adding a salt. In another embodiment, the operating condition further comprises modulating the conductivity of the operating solution by adding a salt. In yet another embodiment, the operating condition further comprises modulating the ionic strength and conductivity of the operating solution by adding a salt. In some embodiments, the effect of adding a salt is to achieve the desired log $\alpha$. In other embodiments, the effect of adding a salt is to achieve the desired log $K_p$ for the lipase. In yet other embodiments, the effect of adding a salt is to achieve the desired log $\alpha$ and the desired log $K_p$ for the lipase.

In some embodiments, the salt in the operating solution is selected from the group consisting of sodium chloride, sodium acetate, sodium phosphate, ammonium sulfate, sodium sulfate, and Tris-HCl. In one embodiment, the salt is sodium chloride. In another embodiment, the salt is sodium acetate. In yet another embodiment, the salt is sodium phosphate. In still another embodiment, the salt is ammonium sulfate. In one embodiment, the salt is sodium sulfate. In another embodiment, the salt is Tris-HCl.

In a specific embodiment, the concentration of sodium chloride in the operating solution is from about 100 mM to about 225 mM, the chromatographic resin is CEX, and the pH of the operating condition is from about 5.0 to about 6.0.

In another specific embodiment, the concentration of sodium chloride in the operating solution is from about 150 mM to about 180 mM, the chromatographic resin is CEX, and the pH of the operating condition is from about 5.0 to about 6.0.

In yet another specific embodiment, the concentration of sodium acetate in the operating solution is from about 100 mM to about 200 mM, the chromatographic resin is AEX; the pH of the operating condition is from about 6.9 to about 7.8.

In still another specific embodiment, the concentration of sodium sulfate in the operating solution is from about 500 mM to about 620 mM, the chromatographic resin is HIC, and the pH of the operating condition is about 7.

In yet still another specific embodiment, the concentration of sodium sulfate in the operating solution is from about 510 mM to about 560 mM, the chromatographic resin is HIC, and the pH of the operating condition is about 7.

In yet another aspect, provided herein is a method of improving PS-80 stability in a production protein formulation, comprising:

(a) passing a load fluid comprising the production protein through a mixed mode AEX resin;
(b) collecting the production protein in a flowthrough; and
(c) formulating the production protein so that the production protein formulation is a PS-80-containing solution;

wherein the pH of the load fluid is from about pH 7.2 to about pH 7.6, and wherein the load fluid does not comprise a salt.

In still another aspect, provided herein is a method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is from about pH 4.9 to about pH 5.3, and wherein the elution solution further comprises from about 120 mM to about 175 mM sodium chloride.

In one embodiment, the method of improving PS-80 stability in a production protein formulation comprises:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is about pH 5.1, and wherein the elution solution further comprises about 150 mM sodium chloride.

In yet another embodiment, the method of improving PS-80 stability in a production protein formulation comprises:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is about pH 5.1, and wherein the elution solution further comprises about 165 mM sodium chloride.

In still another aspect, provided herein is a method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is from about pH 5.0 to about pH 5.4, and wherein the elution solution further comprises from about 150 mM to about 275 mM sodium chloride.

In one embodiment, the method of improving PS-80 stability in a production protein formulation comprises:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is about pH 5.1, and wherein the elution solution further comprises about 200 mM sodium chloride.

In another embodiment, the method of improving PS-80 stability in a production protein formulation comprises:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is about pH 5.1, and wherein the elution solution further comprises about 250 mM sodium chloride.

In more embodiments of the various methods provided herein, the load fluid is an eluate from a prior chromatographic process. In one embodiment, the prior chromatographic process comprises an affinity chromatography. In another embodiment, the prior chromatographic process comprises an affinity chromatography followed by a non-affinity chromatography. In yet another embodiment, the affinity chromatography is a protein A chromatography. In still another embodiment, the non-affinity chromatography is an AEX chromatography. In yet still another embodiment, the prior chromatographic process comprises a protein A chromatography followed by an AEX chromatography.

In yet still another aspect, provided herein is a method of separating a host cell lipase from a production protein through a CEX chromatographic process, comprising:

(a) passing a load fluid comprising the host cell lipase and the production protein through a CEX resin; and
(b) eluting the production protein from the CEX resin with an elution solution;

wherein the pH of the elution solution is from about pH 4.9 to about pH 5.4, and wherein the conductivity of the elution solution is from about 15 mS/cm to about 21 mS/cm.

In another aspect, provided herein is a method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 4.9 to about pH 5.4, and wherein the conductivity of the elution solution is from about 15 mS/cm to about 21 mS/cm.

In yet still another aspect, provided herein is a method of separating a host cell lipase from a production protein through a CEX chromatographic process, comprising:

(a) passing a load fluid comprising the host cell lipase and the production protein through the CEX resin; and
(b) eluting the production protein from the CEX resin with an elution solution;

wherein the pH of the elution solution is from about pH 4.9 to about pH 5.4, and wherein the elution solution further comprises from about 135 mM to about 195 mM sodium chloride.

In another aspect, provided herein is a method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 4.9 to about pH 5.4, and wherein the elution solution further comprises from about 135 mM to about 195 mM sodium chloride.

In certain embodiments of various method described herein, the production protein is a therapeutic protein.

In some embodiments of various method described herein, the production protein is a monoclonal antibody.

In another aspect, provided herein is a pharmaceutical composition comprising a therapeutic protein and less than 1 ppm of a host cell lipase. In some embodiments, the pharmaceutical composition comprises a therapeutic protein and less than 0.1, 0.2, 0.3, 0.4, 0.5, 0.6, 0.7, 0.8, or 0.9 ppm of a host cell lipase. In one embodiment, the pharmaceutical composition comprises a therapeutic protein and less than 0.1 ppm of a host cell lipase. In another embodiment, the pharmaceutical composition comprises a therapeutic protein and less than 0.2 ppm of a host cell lipase. In yet another embodiment, the pharmaceutical composition comprises a therapeutic protein and less than 0.3 ppm of a host cell lipase. In still another embodiment, the pharmaceutical composition comprises a therapeutic protein and less than 0.4 ppm of a host cell lipase. In yet still another embodiment, the pharmaceutical composition comprises a therapeutic protein and less than 0.5 ppm of a host cell lipase. In one embodiment, the pharmaceutical composition comprises a therapeutic protein and less than 0.6 ppm of a host cell lipase. In another embodiment, the pharmaceutical composition comprises a therapeutic protein and less than 0.7 ppm of a host cell lipase. In yet another embodiment, the pharmaceutical composition comprises a therapeutic protein and less than 0.8 ppm of a host cell lipase. In still another embodiment, the pharmaceutical composition comprises a therapeutic protein and less than 0.9 ppm of a host cell lipase.

In various embodiments of the pharmaceutical compositions described herein, the level of the host cell lipase is measured by liquid chromatography-mass spectrometry (LC-MS).

In certain embodiments, the pharmaceutical composition is an eluate from a CEX chromatography using an elution solution selected from the group consisting of:

(a) an elution solution with a pH from about 4.9 to about 5.3, comprising from about 120 mM to about 175 mM sodium chloride;
(b) an elution solution with a pH of about 5.1, comprising about 150 mM sodium chloride;
(c) an elution solution with a pH of about 5.1, comprising about 165 mM sodium chloride;
(d) an elution solution with a pH from about 4.9 to about 5.4 and a conductivity from about 15 mS/cm to about 21 mS/cm;
(e) an elution solution with a pH from about 4.9 to about 5.4, comprising from about 135 mM to about 195 mM sodium chloride;
(f) an elution solution with a pH from about pH 5.0 to about pH 5.4, comprising from about 150 mM to about 275 mM sodium chloride;
(g) an elution solution with a pH of about 5.1, comprising about 200 mM sodium chloride; and
(h) an elution solution with a pH of about 5.1, comprising about 250 mM sodium chloride.

In one embodiment, the pharmaceutical composition is an eluate from a CEX chromatography using an elution solution with a pH from about 4.9 to about 5.3, comprising from about 120 mM to about 175 mM sodium chloride.

In another embodiment, the pharmaceutical composition is an eluate from a CEX chromatography using an elution solution with a pH of about 5.1, comprising about 150 mM sodium chloride.

In yet another embodiment, the pharmaceutical composition is an eluate from a CEX chromatography using an elution solution with a pH of about 5.1, comprising about 165 mM sodium chloride.

In still another embodiment, the pharmaceutical composition is an eluate from a CEX chromatography using an elution solution with a pH from about 4.9 to about 5.4 and a conductivity from about 15 mS/cm to about 21 mS/cm.

In one embodiment, the pharmaceutical composition is an eluate from a CEX chromatography using an elution solution with a pH from about 4.9 to about 5.4, comprising from about 135 mM to about 195 mM sodium chloride.

In another embodiment, the pharmaceutical composition is an eluate from a CEX chromatography using an elution solution with a pH from about pH 5.0 to about pH 5.4, comprising from about 150 mM to about 275 mM sodium chloride.

In still another embodiment, the pharmaceutical composition is an eluate from a CEX chromatography using an elution solution with a pH of about 5.1, comprising about 200 mM sodium chloride.

In yet still another embodiment, the pharmaceutical composition is an eluate from a CEX chromatography using an elution solution with a pH of about 5.1, comprising about 250 mM sodium chloride.

In some embodiments of the pharmaceutical compositions, the CEX chromatography is preceded by an AEX chromatography operated in a flowthrough mode.

In certain embodiments of the pharmaceutical compositions, the lipase is selected from the group consisting of PLBL2, LPL, LPLA2, LP-PLA2, and LAL. In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2 , LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2 , LP-PLA2, and LAL.

In other embodiments of the pharmaceutical compositions, the therapeutic protein is a monoclonal antibody.

## IV. BRIEF DESCRIPTION OF THE DRAWINGS

**FIGS. 1A-1F** show PLBL2 (**FIGS. 1A-1C**) or LPLA2 (**FIGS. 1D-1F**) log $K_P$ values for a range of typical AEX conditions. **FIG. 1A** and **FIG. 1D** show conditions typical for loading in Tris and acetate buffer. **FIG. 1B** and **FIG. 1E** show conditions typical for equilibrating or washing with Tris buffer. **FIG. 1C** and **FIG. 1F** show conditions for equilibrating or washing with phosphate buffer.

FIGS. 2A-2C show PLBL2 (**FIGS. 2A and 2B**) or LPLA2 (FIG. 2C) log $K_P$ values for a range of typical CEX conditions. FIG. 2A and FIG. 2C show conditions for modulation of binding by mainly varying salt. FIG. 2B shows conditions for modulation of binding by mainly varying pH.

FIG. 3 shows PLBL2 or LPLA2 log $K_P$ values for a range of HIC conditions typical for modulation of binding by salt concentration.

FIGS. 4A and 4B show PLBL2 log $K_P$ values for a range of pH and salt conditions typical for multimodal chromatography resins. FIG. 4A shows conditions for a multimodal anion exchanger, Capto adhere. FIG. 4B shows conditions for a multimodal cation exchanger, Capto MMC.

FIG. 5 shows the chromatogram of an AEX process operated in flowthrough mode under the specified condition, indicating that very little mAb3 bound to the AEX resin.

FIGS. 6A-6C show log $\alpha$ separation factor values for mAb3 and PLBL2 (**FIGS. 6A and 6B**) or mAb3 and LPLA2 (FIG. 6C) at a range of pH and salt conditions typical for CEX chromatography. FIGS. 6A and 6C demonstrate optimizing separation conditions where binding is modulated by mainly varying salt concentrations. FIG. 6B demonstrates optimizing conditions where binding is modulated by mainly varying pH. Black boxes represent regions where separation is maximized.

FIG. 7 shows a comparison of log $K_P$ values on a HIC resin for PLBL2, LPLA2, and two different mAbs, mAb2 and mAb3. mAb3 has very similar binding to PLBL2 and LPLA2, but mAb2 is bound much more weakly than mAb3, PLBL2, and LPLA2, offering greater separation potential of PLBL2 and LPLA2 from mAb2 than from mAb3.

FIGS. 8A and 8B show log $\alpha$ values for mAb3 and PLBL2 at typical operating conditions for multimodal chromatography resins. FIG. 8A demonstrates optimizing separation conditions for a multimodal anion exchanger, Capto adhere. FIG. 8B demonstrates optimizing separation conditions for a multimodal cation exchanger, Capto MMC. Black boxes represent regions where separation is maximized.

FIG. 9 shows Pareto chart summarizing the ranked statistical significance of model parameters (factors) for residual HCP (ng/mg).

FIGS. 10A-10C show PS-80 concentration of the placebo, mAb4 AEX pool drug substance (AEXP DS), and mAb4 CEX pool drug substance (CEXP DS) at 5 °C (FIG. 10A), 25 °C (FIG. 10B), and 40 °C (FIG. 10C) over 26 weeks.

FIGS. **11A and 11B** show percentage of PS80 degradation in formulations containing mAb4 purified by two-column chromatography (Protein A and AEX, FIG. 11A) or three-column chromatography (Protein A, AEX, and CEX, FIG. 11B).

FIG. 12 demonstrates that the three-column chromatography is necessary to fully remove residual lipases for mAb4.

## V. DETAILED DESCRIPTION OF THE INVENTION

### 1. Definitions

Certain technical and scientific terms are specifically defined below. Unless specifically defined elsewhere in this document, all other technical and scientific terms used herein have the meaning commonly understood by one of ordinary skill in the art to which this disclosure relates. In case of conflict, the present specification, including definitions, will control.

The term "operating condition," "operation condition," "processing condition," or "process condition," as used exchangeably herein, refers to the condition for operating a chromatographic process. The operating condition can be equilibration condition, loading condition, wash condition, and/or elution condition, etc. The operating condition includes but is not limited to the type of the chromatographic resin, the resin backbone, the resin ligand, the pH of the operating solution, the composition of the operating solution, the concentration of each ingredient of the operating solution, the conductivity of the operating solution, the ionic strength of the operating solution, the cationic strength of the operating solution, the anionic strength of the operating solution, or a combination of two or more above factors.

The term "operating solution" refers to the solution used in operating a chromatographic process. The operating solution can be equilibration solution, loading or feed solution, wash solution, and/or elution solution, etc.

The term "partition coefficient" or "$K_p$," as used herein, refers to the ratio of the concentration of a protein bound to a chromatographic resin (Q) to the concentration of the protein remaining in the solution (C) at equilibrium under a specific operating condition. The partition coefficient for a particular protein can be calculated as follows: $K_p = Q/C$.

The term "separation factor" or "$\alpha$," as used herein, refers to the ratio of the partition coefficient for a first protein ($K_{p, protein 1}$) and the partition coefficient for a second protein ($K_{p, protein 2}$). The separation factor quantifies the selectivity of a chromatographic resin between the two proteins, under a specific operating condition. It can be used to predict the extent of separation of the two proteins through the chromatographic resin under the operating condition. The separation factor between two proteins can be calculated as follows: $\alpha = K_{p, protein 1} / K_{p, protein 2}$; or $\log \alpha = \log K_{p, protein 1} - \log K_{p, protein 2}$.

"Production protein," as used herein, refers to any protein that is the intended product of a bioprocess. Non-limiting examples of the production protein include therapeutic proteins, antibodies (e.g., monoclonal antibodies), hormones, cytokines, enzymes, growth factors, clotting factors, or immunoconjugates thereof, fusion proteins thereof, or fragments thereof.

"Therapeutic protein," as used herein, refers to any protein that has therapeutic effect in an animal (e.g., human, cow, horse, dog, etc.). Non-limiting examples of the therapeutic protein include antibodies (e.g., monoclonal antibodies, bispecific antibodies, or antigen-binding fragments thereof, etc.), hormones, cytokines, enzymes, growth factors, clotting factors, or immunoconjugates thereof, fusion proteins thereof, or fragments thereof.

"Eluate," as used herein, refers to the liquid that passes through a chromatography. In some embodiments, the eluate is the flowthrough of a loading solution. In other embodiments, the eluate comprises the elution solution that passes through the chromatography and any additional components eluted from the chromatography.

"Mixed mode" or "multimodal," when used with a chromatographic resin, means that the resin can separate molecules by more than one mode, function, or mechanism, for example, an ion exchange and a hydrophobic interaction. In some embodiments, the mixed mode or multimodal chromatographic resin can separate molecules by both cation exchange and hydrophobic interaction. In other embodiments, the mixed mode or multimodal chromatographic resin can separate molecules by both anion exchange and hydrophobic interaction.

"Polysorbate-80 stability" or "PS-80 stability," as used herein, refers to the state of PS-80 remaining physically, chemically, and/or biologically stable under common storage conditions (e.g., 5°C $\pm$ 3°C, 25°C $\pm$ 3°C, 60% $\pm$ 5% relative humidity (RH), 40°C $\pm$ 2°C, 75% $\pm$ 5% relative humidity (RH)) over a period of time (e.g., 1 week, 1 month, 6 months, 1 year, 2 years, etc.) The PS-80 stability can be measured by the amount of intact PS-80 molecules and/or the amount of degraded products using various methods, including but not limited to mass spectrometry (MS), liquid chromatography-mass spectrometry (LCMS), or solid phase extraction (SPE) on a HPLC system with a charged aerosol detector (CAD).

"Monoclonal antibody" or "mAb" or "Mab", as used herein, refers to a population of substantially homogeneous antibodies, i.e., the antibody molecules comprising the population are identical in amino acid sequence except for possible naturally occurring mutations that may be present in minor amounts. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present disclosure may be made by the hybridoma method first described by Kohler et al. (1975) Nature 256: 495, or may be made by recombinant DNA methods (see, e.g., U.S. Pat. No. 4,816,567). The "monoclonal antibodies" may also be isolated from phage antibody libraries using the techniques described in Clackson et al. (1991) Nature 352: 624-628 and Marks et al. (1991) J. Mol. Biol. 222: 581-597, for example. See also Presta (2005) J. Allergy Clin. Immunol. 116:731.

"About" when used to modify a numerically defined parameter (e.g., pH, concentration, etc.) means that the parameter is within 20%, within 15%, within 10%, within 9%, within 8%, within 7%, within 6%, within 5%, within 4%, within 3%, within 2%, within 1%, or less of the stated numerical value or range for that parameter; where appropriate, the stated parameter may be rounded to the nearest whole number.

As used herein, including the appended claims, the singular forms of words such as "a," "an," and "the," include their corresponding plural references unless the context clearly dictates otherwise. Unless otherwise required by context, singular terms shall include pluralities and plural terms shall include the singular.

As used herein, the terms "at least one" item or "one or more" item each include a single item selected from the list as well as mixtures of two or more items selected from the list.

Any example(s) following the term "e.g." or "for example" is not meant to be exhaustive or limiting.

Unless expressly stated to the contrary, all ranges cited herein are inclusive; i.e., the range includes the values for the upper and lower limits of the range as well as all values in between. As an example, temperature ranges, percentages, ranges of equivalents, and the like described herein include the upper and lower limits of the range and any value in the continuum there between. All ranges also are intended to include all included sub-ranges, although not necessarily explicitly set forth. For example, a range of pH 4.0-5.0 is intended to include pH 4.0, 4.1, 4.13, 4.2, 4.1-4.6, 4.3-4.4, and 5.0. In addition, the term "or," as used herein, denotes alternatives that may, where appropriate, be combined; that is, the term "or" includes each listed alternative separately as well as their combination.

Where aspects or embodiments of the disclosure are described in terms of a Markush group or other grouping of alternatives, the present disclosure encompasses not only the entire group listed as a whole, but each member of the group individually and all possible subgroups of the main group, but also the main group absent one or more of the group members. The present disclosure also envisages the explicit exclusion of one or more of any of the group members in the claims.

Exemplary methods and materials are described herein, although methods and materials similar or equivalent to those described herein can also be used in the practice or testing of the present disclosure. The materials, methods, and examples are illustrative only and not intended to be limiting.

## 2. Chromatographic Processes

The various methods provided herein can be used with any chromatographic process disclosed herein or understood by a person of ordinary skill in the art, for separation of a production protein from impurities. Non-limiting examples of chromatographic processes include IEX, AEX, CEX, HIC, mixed mode AEX, mixed mode CEX, affinity, and hydroxyapatite chromatographic (HAC) process, etc. In one embodiment, the chromatographic process is an IEX chromatographic process. In another embodiment, the chromatographic process is an AEX chromatographic process. In yet another embodiment, the chromatographic process is a CEX chromatographic process. In still another embodiment, the chromatographic process is a HIC chromatographic process. In one embodiment, the chromatographic process is a mixed mode IEX chromatographic process. In another embodiment, the chromatographic process is a mixed mode AEX chromatographic process. In yet another embodiment, the chromatographic process is a mixed mode CEX chromatographic process. In still another embodiment, the chromatographic process is an affinity chromatographic process. In one embodiment, the chromatographic process is a protein A affinity chromatographic process. In another embodiment, the chromatographic process is a protein G affinity chromatographic process. In yet another embodiment, the chromatographic process is an immobilized metal affinity chromatographic (IMAC) process. In still another embodiment, the chromatographic process is a HAC process.

IEX chromatography separates molecules based on net charge of the molecules. Separation occurs as a result of competition between the charged molecule of interest and counter ions for oppositely charged ligand groups on the IEX chromatographic resin. Strength of the binding of the molecule to the IEX resin depends on the net charge of the molecules, which is affected by operating conditions, such as pH and ionic strength. IEX resins include AEX resins and CEX resins. AEX resins may contain substituents such as diethylaminoethyl (DEAE), trimethylaminoethyl (TMAE), quaternary aminoethyl (QAE) and quaternary amine (O) groups. CEX resins may contain substituents such as carboxymethyl (CM), sulfoethyl (SE), sulfopropyl (SP), phosphate (P) and sulfonate (S). Cellulosic IEX resins such as DE23, DE32, DE52, CM-23, CM-32 and CM-52 are available from Whatman Ltd. Maidstone, Kent, U.K. Sephadex-based and cross-linked IEX resins are also known. For example, DEAE-, QAE-, CM-, and SP-Sephadex, and DEAE-, Q-, CM- and S-Sepharose, and Sepharose are all available from GE Healthcare, Piscataway, NJ. Further, both DEAE and CM derived ethylene glycol-methacrylate copolymer such as TOYOPEARL™ DEAE-650S or M and TOYOPEARL™ CM-650S or M are available from Toso Haas Co., Philadelphia, PA. POROS™ HS, POROS™ HQ, POROS™ XS are available from Thermo Fisher Scientific, Waltham, MA.

HIC chromatography separates molecules based on hydrophobicity of molecules. Hydrophobic regions in the molecule of interest bind to the HIC resin through hydrophobic interaction. Strength of the interaction depends on operating conditions such as pH, ionic strength, and salt concentration. In general, HIC resins contain a base matrix (e.g., cross-linked agarose or synthetic copolymer material) to which hydrophobic ligands (e.g., alkyl or aryl groups) are coupled. Non-limiting examples of HIC resins include Phenyl SEPHAROSE™ 6 FAST FLOW™ (Pharmacia LKB Biotechnology, AB, Sweden); Phenyl SEPHAROSE™ High Performance (Pharmacia LKB Biotechnology, AB, Sweden); Octyl SEPHAROSE™ High Performance (Pharmacia LKB Biotechnology, AB, Sweden); Fractogel™ EMD Propyl or FRACTOGEL™ EMD Phenyl (E. Merck, Germany); MACRO-PREP™ Methyl or MACRO-PREP™ t-Butyl Supports (Bio-Rad, CA); WP HI-Propyl (C$_3$)™ (J. T. Baker, NJ); TOYOPEARL™ ether, phenyl or butyl (TosoHaas, PA); and Tosoh-Butyl-650M (Tosoh Corp., Tokyo, Japan).

HAC chromatography uses an insoluble hydroxylated calcium phosphate of the formula $[Ca_{10}(PO_4)_6(OH)_2]$ as both the matrix and the ligand. The functional groups of the HAC resin include pairs of positively charged calcium ions (C-sites) and negatively charged phosphate groups (P-sites). The C-sites can interact with carboxylate residues on the protein surface while the P-sites can interact with basic protein residues. Strength of the binding between the protein and the HAC resin depends on operating conditions including pH, ionic strength, composition of solution, concentration of each component of the composition, gradient of pH, gradient of component concentration, etc. Various HAC resins, such as CHT™ Ceramic Hydroxyapatite and CFT™ Ceramic Fluoroapatite, are commercially available.

Affinity chromatography separates molecules based on a highly specific interaction between the molecule of interest and the functional group of the resin, such as interaction between antigen and antibody, enzyme and substrate, receptor and ligand, or protein and nucleic acid, etc. Some commonly used affinity chromatographic resins include protein A or protein G resin to purify antibodies, avidin biotin resin to purify biotin/avidin and their derivatives, glutathione resin to purify GST-tagged recombinant proteins, heparin resin to separate plasma coagulation proteins, IMAC resin to purify proteins that specifically interact with the metal ions, etc. Operating conditions of each affinity chromatography depend on the mechanism of the interaction and factors that affect the interaction. Commercial affinity chromatographic resins include but are not limited to MabSelect Sure, UNOsphere SUPrA™, Affi-Gel®, and Affi-Prep®.

In certain embodiments, the chromatographic resin employed herein can separate molecules based on more than one function or mechanism, *i.e.,* in a mixed mode. The mixed mode can be a combination of any two or more functions or mechanisms described above or understood by a person of ordinary skill in the art, such as a combination of IEX and HIC *(e.g.,* AEX/HIC or CEX/HIC), a combination of AEX and CEX (AEX/CEX), or a combination of HIC, AEX, and CEX (HIC/AEX/CEX), etc. Exemplary mixed mode chromatographic resins include but are not limited to OminPac PCX-500, Primesep®, Obelisc R, Oblisc N, Acclaim Trinity P1, Acclaim Trinity P2, Capto Adhere, Capto Adhere Impres, Capto MMC, Capto MMC Impres, Capto Core 700, PPA Hypercel, HEA Hypercel, MEP Hypercel, Eshmuno HCX, Toyopearl MX-Trp-650M, Nuvia C Prime, CHT Type I, and CHT Type II.

### 3. Partition Coefficient ($K_p$) and Separation Factor ($\alpha$)

Partition coefficient ($K_p$) and separation factor ($\alpha$) are two thermodynamic parameters specific for an operating condition of a chromatographic process, which can be used to quantify separation that can be achieved through the process under the operating condition.

A partitioning coefficient, $K_P$, is determined by mixing a known liquid concentration of protein (or other molecule of interest) with a known volume of chromatographic resin and calculating the ratio of the protein bound to the resin and the protein remaining in the liquid at equilibrium: $K_P = q/c = [bound]/[free]$.

Partitioning is generally reported in terms of $\log K_P$, which can be accurately quantified from approximately 0 to 2 using the UV method described herein. General rules for $\log K_P$ screening are as follows:

$$\log K_P \geq 1.5,$$

strong binding to the resin;

$$\log K_P < 1,$$

conditions where elution would be expected for a bind-and-elute modality;

$$0.5 < \log K_P < 1,$$

weak interaction conditions that will show some binding;

$$\log K_P < 0.5,$$

very little or no binding.

The difference of $\log K_p$ values between different species can be used to predict separation of the species through the calculation of a separation factor, $\alpha$, as follows: $\alpha = K_{P, \text{protein 1}}/K_{P, \text{protein 2}}$; $\log \alpha = \log K_{P, \text{protein 1}} - \log K_{P, \text{protein 2}}$, where a $\log \alpha$ further from 0 indicates better separation. In certain embodiments, an absolute value of $\log \alpha$ larger than 0.2 indicates good separation between the two species. In some embodiments, an absolute value of $\log \alpha$ larger than 0.3 indicates good separation between the two species. In other embodiments, an absolute value of $\log \alpha$ larger than 0.5 indicates good separation between the two species. In other embodiments, an absolute value of $\log \alpha$ larger than 1.0 indicates good separation between the two species.

### 4. HCP and Production Proteins

The various methods provided herein apply to a broad variety of HCP as well as a broad variety of production proteins.

The HCP can be any endogenous protein derived from a host cell *(e.g.,* CHO cell) during bioprocessing of a production protein expressed in the host cell. Non-limiting examples of HCP include structural protein, functional protein, secreted protein, enzyme, such as lipase, proteinase, and kinase, etc. In some embodiments, the HCP is a structural protein. In certain embodiments, the HCP is a functional protein. In other embodiments, the HCP is a secreted protein. In yet other embodiment, the HCP is an enzyme. In one embodiment, the HCP is a lipase. In another embodiment, the HCP is a proteinase. In yet another embodiment, the HCP is a kinase.

In certain embodiments, the lipase is selected from the group consisting of PLBL2, LPL, LPLA2, LP-PLA2, and LAL. In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2 , LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2 , LP-PLA2, and LAL.

The host cell can be any cell used for expressing an exogenous protein. Common host cells used in manufacturing of biopharmaceuticals include but are not limited to CHO cell, baby hamster kidney (BHK21) cell, murine myeloma NS0 cell, murine myeloma Sp2/0 cell, human embryonic kidney 293 (HEK293) cell, fibrosarcoma HT-1080 cell, PER.C6 cell, HKB-11 cell, CAP cell, HuH-7 cell, murine C127 cell, and a naturally generated or genetically modified variant thereof. In certain embodiments, the host cell is CHO cell. In some embodiments, the host cell is baby hamster kidney (BHK21) cell. In other embodiments, the host cell is murine myeloma NS0 cell. In yet other embodiments, the host cell is murine myeloma Sp2/0 cell. In still other embodiments, the host cell is human embryonic kidney 293 (HEK293) cell. In certain embodiments, the host cell is fibrosarcoma HT-1080 cell. In some embodiments, the host cell is PER.C6 cell. In other embodiments, the host cell is HKB-11 cell. In yet other embodiments, the host cell is CAP cell. In still other embodiments, the host cell is HuH-7 cell. In certain embodiments, the host cell is murine C127 cell. In some embodiments, the host cell is a naturally generated variant of the above host cell. In other embodiments, the host cell is a genetically modified variant of the above host cell.

In certain embodiments, the CHO cell lipase is selected from the group consisting of PLBL2, LPL, LPLA2, LP-PLA2, and LAL. In one embodiment, the CHO cell lipase is PLBL2. In another embodiment, the CHO cell lipase is LPL. In yet another embodiment, the CHO cell lipase is LPLA2. In one embodiment, the CHO cell lipase is LP-PLA2. In another embodiment, the CHO cell lipase is LAL. In still another embodiment, the CHO cell lipase includes two, three, four, five, six, seven, eight, nine, ten, or more different CHO cell lipases. In yet still another embodiment, the CHO cell lipase includes two, three, four, or five different CHO cell lipases selected from the group consisting of PLBL2, LPL, LPLA2 , LP-PLA2, and LAL. In one embodiment, the CHO cell lipase includes PLBL2 and LPL. In another embodiment, the CHO cell lipase includes PLBL2 and LPLA2. In yet another embodiment, the CHO cell lipase includes PLBL2 and LP-PLA2. In still another embodiment, the CHO cell lipase includes PLBL2 and LAL. In one embodiment, the CHO cell lipase includes LPL and LPLA2. In another embodiment, the CHO cell lipase includes LPL and LP-PLA2. In yet another embodiment, the CHO cell lipase includes LPL and LAL. In still another embodiment, the CHO cell lipase includes LPLA2 and LP-PLA2. In one embodiment, the CHO cell lipase includes LPLA2 and LAL. In another embodiment, the CHO cell lipase includes LP-PLA2 and LAL. In yet another embodiment, the CHO cell lipase includes PLBL2, LPL, and LPLA2. In still another embodiment, the CHO cell lipase includes PLBL2, LPL, and LP-PLA2. In one embodiment, the CHO cell lipase includes PLBL2, LPL, and LAL. In another embodiment, the CHO cell lipase includes PLBL2, LPLA2, and LP-PLA2. In yet another embodiment, the CHO cell lipase includes PLBL2, LPLA2, and LAL. In still another embodiment, the CHO cell lipase includes PLBL2, LP-PLA2, and LAL. In one embodiment, the CHO cell lipase includes LPL, LPLA2, and LP-PLA2. In another embodiment, the CHO cell lipase includes LPL, LPLA2, and LAL. In yet another embodiment, the CHO cell lipase includes LPL, LP-PLA2, and LAL. In still another embodiment, the CHO cell lipase includes LPLA2, LP-PLA2, and LAL. In one embodiment, the CHO cell lipase includes PLBL2, LPL, LPLA2, and LP-PLA2. In another embodiment, the CHO cell lipase includes PLBL2, LPL, LPLA2, and LAL. In yet another embodiment, the CHO cell lipase includes PLBL2, LPL, LP-PLA2, and LAL. In still another embodiment, the CHO cell lipase includes PLBL2, LPLA2, LP-PLA2, and LAL. In yet still another embodiment, the CHO cell lipase includes PLBL2, LPL, LPLA2 , LP-PLA2, and LAL.

The production protein can be any protein of interest expressed in the host cell for the purpose of generating a biopharmaceutical product. Non-limiting examples of production proteins include therapeutic proteins, monoclonal antibodies, hormones, cytokines, growth factors, clotting factors, enzymes, fusion proteins thereof, immunoconjugates thereof, and fragments thereof. In certain embodiments, the production protein is a therapeutic protein. In some embodiments, the production protein is a monoclonal antibody. In other embodiments, the production protein is a hormone. In yet other embodiments, the production protein is a cytokine. In still other embodiments, the production protein is a growth factor. In certain embodiments, the production protein is a clotting factor. In some embodiments, the production protein is an enzyme. In other embodiments, the production protein is a fusion protein of the above production protein. In yet other embodiments, the production protein is an immunoconjugate of the above production protein. In still other embodiments, the production protein is a fragment of the above production protein.

In some embodiments, the production protein is a monoclonal antibody specific for an antigen including but not limited to PD-1, PD-L1, CTLA-4, LAG3, TIM3, TIGIT, GITR, TNF-$\alpha$, HER2, GPIIb/IIIa, CD52, PCSK9, IL-2R$\alpha$, BLyS, VEGF, clostridium difficile toxin B, CD19, CD30, IL-1$\beta$, IL17R$\alpha$, PSMA, EGFR, IL-2R, IL-2R$\beta\gamma$, CD38, RANKL, GD2, IL-4R$\alpha$, complement component 5, CD20, SLAMF7, dabigatran, IL-5, $\alpha$-4 integrin, PDGFR$\alpha$, VEGFR1, VEGFR2, F protein of RSV, IL-6, IL-6R, IL-12, IL-23, and CD33. In one embodiment, the production protein is an anti-PD-1 monoclonal antibody. In another embodiment, the production protein is an anti-CTLA-4 monoclonal antibody. In yet another embodiment, the production protein is an anti-LAG3 monoclonal antibody. In still another embodiment, the production protein is an anti-TIGIT monoclonal antibody. In one embodiment, the production protein is an anti-GITR monoclonal antibody.

In a specific embodiment, the anti-PD-1 monoclonal antibody is pembrolizumab. In another embodiment, the anti-PD-1 monoclonal antibody is nivolumab. In yet another embodiment, the anti-PD-1 monoclonal antibody is pidilizumab (U.S. Pat. No. 7,332,582). In still another embodiment, the anti- PD-1 monoclonal antibody is AMP-514 (MedImmune LLC, Gaithersburg, MD). In another embodiment, the anti- PD-1 monoclonal antibody is PDR001 (U.S. Pat. No. 9,683,048). In yet another embodiment, the anti- PD-1 monoclonal antibody is BGB-A317 (U.S. Pat. No. 8,735,553). In still another embodiment, the anti- PD-1 monoclonal antibody is MGA012 (MacroGenics, Rockville, MD).

In one embodiment, the anti- LAG3 monoclonal antibody is BMS-986016 (Bristol-Myers Squibb, New York, NY). In another embodiment, the anti- LAG3 monoclonal antibody is REGN3767 (Regeneron, Tarrytown, NY). In yet another embodiment, the anti- LAG3 monoclonal antibody is LAG525 (Novartis, Basel, Switzerland). In still another embodiment, the anti- LAG3 monoclonal antibody is GSK2813781 (GlaxoSmithKline, Brentford, UK).

In one embodiment, the anti- TIGIT monoclonal antibody is BMS-986207 (Bristol-Myers Squibb, New York, NY). In another embodiment, the anti- TIGIT monoclonal antibody is OMP-313M32 (OncoMed Pharmaceuticals, Redwood city, CA). In yet another embodiment, the anti- TIGIT monoclonal antibody is MTIG7192A (also known as RG6058, U.S. Publ. No. 2017/0088613). In still another embodiment, the anti- TIGIT monoclonal antibody is PTZ-201 (Potenza Therapeutics, Cambridge, MA; also known as ASP8374, Astellas Pharma, Tokyo, Japan).

## 5. Methods of Screening Operating Conditions for Separation of a Host Cell Lipase from a Production Protein

This disclosure provides methods of screening operating conditions for separation of a HCP (*e.g.,* lipase) from a production protein (*e.g.,* monoclonal antibody) through a chromatographic process.

Screening can be done by batch binding studies, mini-column binding studies, or any other methods that one of ordinary skill in the art would understand. A plethora of combinations of chromatographic resins and operating conditions, including pH, with or without salt, salt type, salt concentration, other components (*e.g.*, counter ion) in solution, concentration of each component, or load protein concentration, etc., can be designed and examined for the HCP (*e.g.,* lipase) or the production protein (*e.g.,* monoclonal antibody). The $K_p$ values of the HCP (*e.g.,* lipase) and the production protein (*e.g.*, monoclonal antibody) are determined by methods disclosed herein or commonly understood by a person of ordinary skill in the art. Log $\alpha$ values between the HCP (*e.g.,* lipase) and the production protein (*e.g.,* monoclonal antibody) are calculated using methods described herein. In general, an absolute value of log $\alpha$ larger than 0.5 is desirable for good separation between the HCP (*e.g.,* lipase) and the production protein (*e.g.,* monoclonal antibody).

Chromatographic resins to be screened can be any chromatographic resins that may separate the HCP (*e.g.,* lipase) from the production protein (*e.g.,* monoclonal antibody) based on characteristics of the HCP (*e.g.,* lipase) and the production protein (*e.g.,* monoclonal antibody). Operating conditions to be screened can be commonly used process conditions for each resin selected, for example, equilibration condition, loading condition, washing condition, elution condition, or stripping condition, etc.

In one embodiment, the screening is performed using a resin slurry plate method, as disclosed in Welsh et al., Biotechnol Prog. 30 (3):626-635 (2014). For example, mixtures of different combinations of pH, salt, and feed are added into 96-well filter plates (*e.g.*, P/N MSBVN1250, Millipore Sigma, Burlington, MA). The chromatographic resin volume is 2-50 $\mu$L, and the liquid feed volume is 200 $\mu$L. In some embodiments, 16-32 conditions are tested for each resin. In other embodiments, 24-96 conditions are tested for each resin. Separation of resin and liquid was accomplished by vacuum filtration. First, the resin is incubated with the equilibration buffer for 10 minutes and the equilibration step is repeated three times. Next, the resin is incubated with feed for 60 minutes. Then, the resin is incubated in strip condition for 10 minutes and repeated twice. The equilibration step allows for buffer exchange from the initial resin slurry buffer. The 60 min time for feed mixing allows for pseudo equilibration between the resin ligand and protein at a given set of conditions. The filtrate from the feed step was measured by UV absorbance at 280 - 320 nm to determine the final liquid concentration of the protein, c. The bound concentration of the protein, q, was determined by a mass balance of c and the known feed concentration, $c_0$.

In another embodiment, the screening is performed using a mini-column method, as disclosed in Welsh et al., Biotechnol Prog. 30 (3):626-635 (2014) or Petroff et al., Biotech Bioeng. 113 (6):1273-1283 (2015). For example, mixtures of different combinations of pH, salt, and feed are screened in a 0.6 mL column format with a 3 cm bed height. Up to 8 columns are screened in parallel. A typical residence time of about 4 min is preserved in the miniature columns by reducing the linear flowrate from about 300 cm/h for a typical column to about 45 cm/h in the miniature column format. All other typical parameters for chromatography screening are conserved. Eluate factions can be collected as pools or as fractions by collecting in 96-well plates to produce chromatograms similar to lab scale studies.

Once the operating conditions for separating the HCP (e.g., lipase) from the production protein (e.g., monoclonal antibody) are determined, the conditions of the load fluid and/or resin can be adjusted accordingly. For example, the resin can be equilibrated by washing it with a solution that will bring it to the necessary operating conditions.

## 6. Methods of Separating a Host Cell Lipase from a Production Protein

This disclosure further provides methods of separating a HCP (e.g., lipase) from a production protein (e.g., monoclonal antibody) through a chromatographic process.

In one aspect, provided herein is a method of separating a host cell lipase from a production protein through a chromatographic process, comprising:

(a) passing a load fluid comprising the lipase and the production protein through a chromatographic resin under a loading operating condition; and
(b) collecting the production protein in a flowthrough;

wherein separation factor ($\alpha$) is the ratio of the partition coefficient ($K_p$) for the lipase to the $K_p$ for the production protein, and wherein log $\alpha$ is larger than 0.5 under the loading operating condition.

In certain embodiments, log $\alpha$ is larger than 1.0 under the loading operating condition.

In some embodiments, the log $K_p$ for the lipase is larger than 1.0 under the loading operating condition. In other embodiments, the log $K_p$ for the lipase is larger than 1.5 under the loading operating condition.

In certain embodiments, log $\alpha$ is larger than 0.5 and the log $K_p$ for the lipase is larger than 1.0 under the loading operating condition. In some embodiments, log $\alpha$ is larger than 0.5 and the log $K_p$ for the lipase is larger than 1.5 under the loading operating condition. In other embodiments, log $\alpha$ is larger than 1.0 and the log $K_p$ for the lipase is larger than 1.0 under the loading operating condition. In yet other embodiments, log $\alpha$ is larger than 1.0 and the log $K_p$ for the lipase is larger than 1.5 under the loading operating condition.

In another aspect, provided herein is a method of separating a host cell lipase from a production protein through a chromatographic process, comprising:

(a) passing a load fluid comprising the lipase and the production protein through a chromatographic resin; and
(b) eluting the production protein from the chromatographic resin with an elution solution under an elution operating condition;

wherein $\alpha$ is the ratio of $K_p$ for the lipase to the $K_p$ for the production protein, and wherein log $\alpha$ is larger than 0.5 under the elution operating condition.

In certain embodiments, log $\alpha$ is larger than 1.0 under the elution operating condition.

In some embodiments, the log $K_p$ for the lipase is larger than 1.0 under the elution operating condition. In other embodiments, the log $K_p$ for the lipase is larger than 1.5 under the elution operating condition.

In certain embodiments, log $\alpha$ is larger than 0.5 and the log $K_p$ for the lipase is larger than 1.0 under the elution operating condition. In some embodiments, log $\alpha$ is larger than 0.5 and the log $K_p$ for the lipase is larger than 1.5 under the elution operating condition. In other embodiments, log $\alpha$ is larger than 1.0 and the log $K_p$ for the lipase is larger than 1.0 under the elution operating condition. In yet other embodiments, log $\alpha$ is larger than 1.0 and the log $K_p$ for the lipase is larger than 1.5 under the elution operating condition.

In some embodiments of various methods provided herein, the lipase is a CHO cell lipase.

In certain embodiments, the lipase is selected from the group consisting of PLBL2, LPL, LPLA2, LP-PLA2, and LAL. In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2 , LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2 , LP-PLA2, and LAL.

In certain embodiments, the CHO cell lipase is selected from the group consisting of PLBL2, LPL, LPLA2, LP-PLA2, and LAL. In one embodiment, the CHO cell lipase is PLBL2. In another embodiment, the CHO cell lipase is LPL. In yet another embodiment, the CHO cell lipase is LPLA2. In one embodiment, the CHO cell lipase is LP-PLA2. In another embodiment, the CHO cell lipase is LAL. In still another embodiment, the CHO cell lipase includes two, three, four, five, six, seven, eight, nine, ten, or more different CHO cell lipases. In yet still another embodiment, the CHO cell lipase includes two, three, four, or five different CHO cell lipases selected from the group consisting of PLBL2, LPL, LPLA2 , LP-PLA2, and LAL. In one embodiment, the CHO cell lipase includes PLBL2 and LPL. In another embodiment, the CHO cell lipase includes PLBL2 and LPLA2. In yet another embodiment, the CHO cell lipase includes PLBL2 and LP-PLA2. In still another embodiment, the CHO cell lipase includes PLBL2 and LAL. In one embodiment, the CHO cell lipase includes LPL and LPLA2. In another embodiment, the CHO cell lipase includes LPL and LP-PLA2. In yet another embodiment, the CHO cell lipase includes LPL and LAL. In still another embodiment, the CHO cell lipase includes LPLA2 and LP-PLA2. In one embodiment, the CHO cell lipase includes LPLA2 and LAL. In another embodiment, the CHO cell lipase includes LP-PLA2 and LAL. In yet another embodiment, the CHO cell lipase includes PLBL2, LPL, and LPLA2. In still another embodiment, the CHO cell lipase includes PLBL2, LPL, and LP-PLA2. In one embodiment, the CHO cell lipase includes PLBL2, LPL, and LAL. In another embodiment, the CHO cell lipase includes PLBL2, LPLA2, and LP-PLA2. In yet another embodiment, the CHO cell lipase includes PLBL2, LPLA2, and LAL. In still another embodiment, the CHO cell lipase includes PLBL2, LP-PLA2, and LAL. In one embodiment, the CHO cell lipase includes LPL, LPLA2, and LP-PLA2. In another embodiment, the CHO cell lipase includes LPL, LPLA2, and LAL. In yet another embodiment, the CHO cell lipase includes LPL, LP-PLA2, and LAL. In still another embodiment, the CHO cell lipase includes LPLA2, LP-PLA2, and LAL. In one embodiment, the CHO cell lipase includes PLBL2, LPL, LPLA2, and LP-PLA2. In another embodiment, the CHO cell lipase includes PLBL2, LPL, LPLA2, and LAL. In yet another embodiment, the CHO cell lipase includes PLBL2, LPL, LP-PLA2, and LAL. In still another embodiment, the CHO cell lipase includes PLBL2, LPLA2, LP-PLA2, and LAL. In yet still another embodiment, the CHO cell lipase includes PLBL2, LPL, LPLA2 , LP-PLA2, and LAL.

In some embodiments, the production protein is a therapeutic protein. In certain embodiment, the production protein is a monoclonal antibody. In some embodiments, the production protein is a monoclonal antibody specific for an antigen including but not limited to PD-1, PD-L1, CTLA-4, LAG3, TIM3, TIGIT, GITR, TNF-$\alpha$, HER2, GPIIb/IIIa, CD52, PCSK9, IL-2R$\alpha$, BLyS, VEGF, clostridium difficile toxin B, CD19, CD30, IL-1$\beta$, IL17R$\alpha$, PSMA, EGFR, IL-2R, IL-2R$\beta\gamma$, CD38, RANKL, GD2, IL-4R$\alpha$, complement component 5, CD20, SLAMF7, dabigatran, IL-5, $\alpha$-4 integrin, PDGFR$\alpha$, VEGFR1, VEGFR2, F protein of RSV, IL-6, IL-6R, IL-12, IL-23, and CD33. In one embodiment, the production protein is an anti-PD-1 monoclonal antibody. In another embodiment, the production protein is an anti-CTLA-4 monoclonal antibody. In yet another embodiment, the production protein is an anti-LAG3 monoclonal antibody. In still another embodiment, the production protein is an anti-TIGIT monoclonal antibody. In one embodiment, the production protein is an anti-GITR monoclonal antibody.

In a specific embodiment, the anti-PD-1 monoclonal antibody is pembrolizumab. In another embodiment, the anti-PD-1 monoclonal antibody is nivolumab. In yet another embodiment, the anti-PD-1 monoclonal antibody is pidilizumab (U.S. Pat. No. 7,332,582). In still another embodiment, the anti- PD-1 monoclonal antibody is AMP-514 (MedImmune LLC, Gaithersburg, MD). In another embodiment, the anti- PD-1 monoclonal antibody is PDR001 (U.S. Pat. No. 9,683,048). In yet another embodiment, the anti- PD-1 monoclonal antibody is BGB-A317 (U.S. Pat. No. 8,735,553). In still another embodiment, the anti- PD-1 monoclonal antibody is MGA012 (MacroGenics, Rockville, MD).

In one embodiment, the anti- LAG3 monoclonal antibody is BMS-986016 (Bristol-Myers Squibb, New York, NY).

In another embodiment, the anti- LAG3 monoclonal antibody is REGN3767 (Regeneron, Tarrytown, NY). In yet another embodiment, the anti- LAG3 monoclonal antibody is LAG525 (Novartis, Basel, Switzerland). In still another embodiment, the anti- LAG3 monoclonal antibody is GSK2813781 (GlaxoSmithKline, Brentford, UK).

In one embodiment, the anti- TIGIT monoclonal antibody is BMS-986207 (Bristol-Myers Squibb, New York, NY). In another embodiment, the anti- TIGIT monoclonal antibody is OMP-313M32 (OncoMed Pharmaceuticals, Redwood city, CA). In yet another embodiment, the anti- TIGIT monoclonal antibody is MTIG7192A (also known as RG6058, U.S. Publ. No. 2017/0088613). In still another embodiment, the anti- TIGIT monoclonal antibody is PTZ-201 (Potenza Therapeutics, Cambridge, MA; also known as ASP8374, Astellas Pharma, Tokyo, Japan).

In some embodiments of various methods provided herein, the chromatographic resin is an IEX resin. In other embodiments, the chromatographic resin is a HIC resin. In one embodiment, the IEX resin is a CEX resin. In another embodiment, the CEX resin is a mixed mode CEX resin. In yet another embodiment, the IEX resin is an AEX resin. In still another embodiment, the AEX resin is a mixed mode AEX resin.

In certain embodiments of various methods using a CEX resin, the pH of the operating condition is below about 6.0. In some embodiments, the pH of the operating condition is below about 5.5. In other embodiments, the pH of the operating condition is below about 5.0. In yet other embodiments, the pH of the operating condition is from about 4.5 to about 5.5. In still other embodiments, the pH of the operating condition is from about 4.5 to about 5.0. In certain embodiments, the pH of the operating condition is from about 5.0 to about 5.5. In some embodiments, the pH of the operating condition is from about 4.9 to about 5.3.

In certain embodiments of various methods using a mixed mode CEX resin, the pH of the operating condition is below about 6.0. In some embodiments, the pH of the operating condition is below about 5.5. In other embodiments, the pH of the operating condition is below about 5.0. In yet other embodiments, the pH of the operating condition is from about 4.5 to about 5.5. In still other embodiments, the pH of the operating condition is from about 4.5 to about 5.0. In certain embodiments, the pH of the operating condition is from about 5.0 to about 5.5. In some embodiments, the pH of the operating condition is from about 4.9 to about 5.3.

In certain embodiments of various methods using an AEX resin, the pH of the operating condition is above about 6.5. In some embodiments, the pH of the operating condition is above about 6.9. In other embodiments, the pH of the operating condition is above about 7.2. In yet other embodiments, the pH of the operating condition is from about 6.9 to about 7.9. In still other embodiments, the pH of the operating condition is from about 7.2 to about 7.5. In certain embodiments, the pH of the operating condition is from about 7.5 to about 7.8.

In certain embodiments of various methods using a mixed mode AEX resin, the pH of the operating condition is above about 6.5. In some embodiments, the pH of the operating condition is above about 6.9. In other embodiments, the pH of the operating condition is above about 7.2. In yet other embodiments, the pH of the operating condition is from about 6.9 to about 7.9. In still other embodiments, the pH of the operating condition is from about 7.2 to about 7.5. In certain embodiments, the pH of the operating condition is from about 7.5 to about 7.8.

In certain embodiments of various methods provided herein, the operating condition further comprises modulating ionic strength and/or conductivity by adding a salt. In one embodiment, the operating condition further comprises modulating ionic strength by adding a salt. In another embodiment, the operating condition further comprises modulating conductivity by adding a salt. In yet another embodiment, the operating condition further comprises modulating ionic strength and conductivity by adding a salt. In some embodiments, the effect of adding a salt is to achieve the desired log $\alpha$. In other embodiments, the effect of adding a salt is to achieve the desired log $K_p$ for the lipase. In yet other embodiments, the effect of adding a salt is to achieve the desired log $\alpha$ and the desired log $K_p$ for the lipase. Thus, in one embodiment, the operating condition further comprises achieving the desired log $\alpha$ by adding a salt. In another embodiment, the operating condition further comprises achieving the desired log $K_p$ for the lipase by adding a salt. In yet another embodiment, the operating condition further comprises achieving the desired log $\alpha$ and the desired log $K_p$ for the lipase by adding a salt.

In some embodiments, the salt in the operating solution is selected from the group consisting of sodium chloride, sodium acetate, sodium phosphate, ammonium sulfate, sodium sulfate, and Tris-HCl. In one embodiment, the salt is sodium chloride. In another embodiment, the salt is sodium acetate. In yet another embodiment, the salt is sodium phosphate. In still another embodiment, the salt is ammonium sulfate. In one embodiment, the salt is sodium sulfate. In another embodiment, the salt is Tris-HCl.

In a specific embodiment, the concentration of sodium chloride in the operating solution is from about 100 mM to about 225 mM, the chromatographic resin is CEX, and the pH of the operating condition is from about 5.0 to about 6.0.

In another specific embodiment, the concentration of sodium chloride in the operating solution is from about 150 mM to about 180 mM, the chromatographic resin is CEX, and the pH of the operating condition is from about 5.0 to about 6.0.

In yet another specific embodiment, the concentration of sodium acetate in the operating solution is from about 100 mM to about 200 mM, the chromatographic resin is AEX, and the pH of the operating condition is from about 6.9 to about 7.8.

In still another specific embodiment, the concentration of sodium sulfate in the operating solution is from about 500 mM to about 620 mM, the chromatographic resin is HIC, and the pH of the operating condition is about 7.

In yet still another specific embodiment, the concentration of sodium sulfate in the operating solution is from about 510 mM to about 560 mM, the chromatographic resin is HIC, and the pH of the operating condition is about 7.

In yet another aspect, provided herein is a method of separating PLBL2 from a production protein through a mixed mode AEX chromatographic process, comprising:

    (a) passing a load fluid comprising PLBL2 and the production protein through a mixed mode AEX resin; and
    (b) collecting the production protein in a flowthrough;

wherein the pH of the load fluid is from about pH 7.2 to about pH 7.6, and wherein the load fluid does not comprise a salt.

In certain embodiments of such methods, the production protein is a therapeutic protein.

In some embodiments of such methods, the production protein is a monoclonal antibody.

In still another aspect, provided herein is a method of separating PLBL2 from a production protein through a CEX chromatographic process, comprising:

    (a) passing a load fluid comprising PLBL2 and the production protein through a CEX resin; and
    (b) eluting the production protein from the CEX resin with an elution solution;

wherein the pH of the elution solution is from about pH 4.9 to about pH 5.3, and wherein the elution solution further comprises from about 120 mM to about 175 mM sodium chloride.

In certain embodiments of such methods, the production protein is a therapeutic protein.

In some embodiments of such methods, the production protein is a monoclonal antibody.

In one embodiment, the method of separating PLBL2 from a production protein through a CEX chromatographic process comprises:

    (a) passing a load fluid comprising PLBL2 and the production protein through a CEX resin; and
    (b) eluting the production protein from the CEX resin with an elution solution;

wherein the pH of the elution solution is about pH 5.1, and wherein the elution solution further comprises about 150 mM sodium chloride.

In yet another embodiment, the method of separating PLBL2 from a production protein through a CEX chromatographic process comprises:

    (a) passing a load fluid comprising PLBL2 and the production protein through a CEX resin; and
    (b) eluting the production protein from the CEX resin with an elution solution;

wherein the pH of the elution solution is about pH 5.1, and wherein the elution solution further comprises about 165 mM sodium chloride.

In still another aspect, provided herein is a method of separating LPLA2 from a production protein through a CEX chromatographic process, comprising:

    (a) passing a load fluid comprising LPLA2 and the production protein through a CEX resin; and
    (b) eluting the production protein from the CEX resin with an elution solution;

wherein the pH of the elution solution is from about pH 5.0 to about pH 5.4, and wherein the elution solution further comprises from about 150 mM to about 275 mM sodium chloride.

In one embodiment, the method of separating LPLA2 from a production protein through a CEX chromatographic process comprises:

    (a) passing a load fluid comprising LPLA2 and the production protein through a CEX resin; and
    (b) eluting the production protein from the CEX resin with an elution solution;

wherein the pH of the elution solution is about pH 5.1, and wherein the elution solution further comprises about 150 mM sodium chloride.

In another embodiment, the method of separating LPLA2 from a production protein through a CEX chromatographic process comprises:

    (a) passing a load fluid comprising LPLA2 and the production protein through a CEX resin; and

(b) eluting the production protein from the CEX resin with an elution solution;

wherein the pH of the elution solution is about pH 5.1, and wherein the elution solution further comprises about 200 mM sodium chloride.

In yet another embodiment, the method of separating LPLA2 from a production protein through a CEX chromatographic process comprises:

(a) passing a load fluid comprising LPLA2 and the production protein through a CEX resin; and
(b) eluting the production protein from the CEX resin with an elution solution;

wherein the pH of the elution solution is about pH 5.1, and wherein the elution solution further comprises about 250 mM sodium chloride.

In certain embodiments of such a method, the production protein is a therapeutic protein.

In other embodiments of such a method, the production protein is a monoclonal antibody.

The methods of separation provided herein can be used in combination with one or more separation steps described herein or commonly used in the art. In one embodiment, one or more separation steps precede the method described herein. In another embodiment, one or more separation steps follow the method described herein. In yet another embodiment, one or more separation steps are performed between two methods described herein. In still other embodiments, one or more separation steps are performed before, after, and/or between the methods described herein. There is no limitation of how many separation steps or methods can be combined or the order of the separation steps or methods to be combined.

In more embodiments of the various methods provided herein, the load fluid is an eluate from a prior chromatographic process. In one embodiment, the prior chromatographic process comprises an affinity chromatography. In another embodiment, the prior chromatographic process comprises an affinity chromatography followed by a non-affinity chromatography. In yet another embodiment, the affinity chromatography is a protein A chromatography. In still another embodiment, the non-affinity chromatography is an AEX chromatography. In yet still another embodiment, the prior chromatographic process comprises a protein A chromatography followed by an AEX chromatography.

In yet still another aspect, provided herein is a method of separating a host cell lipase from a production protein through a CEX chromatographic process, comprising:

(a) passing a load fluid comprising the host cell lipase and the production protein through a CEX resin; and
(b) eluting the production protein from the CEX resin with an elution solution;

wherein the pH of the elution solution is from about pH 4.9 to about pH 5.4, and wherein the conductivity of the elution solution is from about 15 mS/cm to about 21 mS/cm.

In certain embodiments of such a method, the production protein is a therapeutic protein.

In other embodiments of such a method, the production protein is a monoclonal antibody.

In still another aspect, provided herein is a method of separating a host cell lipase from a production protein through a CEX chromatographic process, comprising:

(a) passing a load fluid comprising the host cell lipase and the production protein through a CEX resin; and
(b) eluting the production protein from the CEX resin with an elution solution;

wherein the pH of the elution solution is from about pH 4.9 to about pH 5.4, and wherein the elution solution further comprises from about 135 mM to about 195 mM sodium chloride.

In certain embodiments of such a method, the production protein is a therapeutic protein.

In other embodiments of such a method, the production protein is a monoclonal antibody.

**7. Methods** of Improving **PS-80 Stability in a Production Protein Formulation**

This disclosure further provides methods of improving PS-80 stability in a production protein formulation (e.g., drug substance formulation or drug product formulation) by separating a HCP *(e.g.,* lipase) from the production protein *(e.g.,* monoclonal antibody) using a chromatographic process.

In yet still another aspect, provided herein is a method of improving PS-80 stability in a production protein formulation, comprising:

(a) passing a load fluid comprising a host cell lipase and the production protein through a chromatographic resin under a loading operating condition;

(b) collecting the production protein in a flowthrough; and

(c) formulating the production protein so that the production protein formulation is a PS-80-containing formulation;

wherein separation factor ($\alpha$) is the ratio of the partition coefficient ($K_p$) for the lipase to the $K_p$ for the production protein, and wherein log $\alpha$ is larger than 0.5 under the loading operating condition.

In certain embodiments, log $\alpha$ is larger than 1.0 under the loading operating condition.

In some embodiments, the log $K_p$ for the lipase is larger than 1.0 under the loading operating condition. In other embodiments, the log $K_p$ for the lipase is larger than 1.5 under the loading operating condition.

In certain embodiments, log $\alpha$ is larger than 0.5 and the log $K_p$ for the lipase is larger than 1.0 under the loading operating condition. In some embodiments, log $\alpha$ is larger than 0.5 and the log $K_p$ for the lipase is larger than 1.5 under the loading operating condition. In other embodiments, log $\alpha$ is larger than 1.0 and the log $K_p$ for the lipase is larger than 1.0 under the loading operating condition. In yet other embodiments, log $\alpha$ is larger than 1.0 and the log $K_p$ for the lipase is larger than 1.5 under the loading operating condition.

In another aspect, provided herein is a method of improving PS-80 stability in a production protein formulation, comprising:

(a) passing a load fluid comprising a host cell lipase and the production protein through a chromatographic resin;

(b) eluting the production protein from the chromatographic resin with an elution solution under an elution operating condition; and

(c) formulating the production protein so that the production protein formulation is a PS-80-containing solution;

wherein $\alpha$ is the ratio of $K_p$ for the lipase to the $K_p$ for the production protein, and wherein log $\alpha$ is larger than 0.5 under the elution operating condition.

In certain embodiments, log $\alpha$ is larger than 1.0 under the elution operating condition.

In some embodiments, the log $K_p$ for the lipase is larger than 1.0 under the elution operating condition. In other embodiments, the log $K_p$ for the lipase is larger than 1.5 under the elution operating condition.

In certain embodiments, log $\alpha$ is larger than 0.5 and the log $K_p$ for the lipase is larger than 1.0 under the elution operating condition. In some embodiments, log $\alpha$ is larger than 0.5 and the log $K_p$ for the lipase is larger than 1.5 under the elution operating condition. In other embodiments, log $\alpha$ is larger than 1.0 and the log $K_p$ for the lipase is larger than 1.0 under the elution operating condition. In yet other embodiments, log $\alpha$ is larger than 1.0 and the log $K_p$ for the lipase is larger than 1.5 under the elution operating condition.

In some embodiments of various methods provided herein, the lipase is a Chinese Hamster Ovary (CHO) cell lipase.

In certain embodiments, the lipase is selected from the group consisting of PLBL2, LPL, LPLA2, LP-PLA2, and LAL. In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2 , LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2 , LP-PLA2, and LAL.

In certain embodiments, the CHO cell lipase is selected from the group consisting of PLBL2, LPL, LPLA2, LP-PLA2, and LAL. In one embodiment, the CHO cell lipase is PLBL2. In another embodiment, the CHO cell lipase is LPL. In yet another embodiment, the CHO cell lipase is LPLA2. In one embodiment, the CHO cell lipase is LP-PLA2. In another embodiment, the CHO cell lipase is LAL. In still another embodiment, the CHO cell lipase includes two, three, four, five, six, seven, eight, nine, ten, or more different CHO cell lipases. In yet still another embodiment, the CHO cell lipase includes two, three, four, or five different CHO cell lipases selected from the group consisting of PLBL2, LPL, LPLA2 , LP-PLA2, and LAL. In one embodiment, the CHO cell lipase includes PLBL2 and LPL. In another embodiment, the CHO cell lipase includes PLBL2 and LPLA2. In yet another embodiment, the CHO cell lipase includes PLBL2 and LP-PLA2. In still another embodiment, the CHO cell lipase includes PLBL2 and LAL. In one embodiment, the CHO cell lipase includes LPL and LPLA2. In another embodiment, the CHO cell lipase includes LPL and LP-PLA2. In yet another embodiment, the CHO cell lipase includes LPL and LAL. In still another embodiment, the CHO cell lipase includes LPLA2 and LP-PLA2. In one embodiment, the CHO cell lipase includes LPLA2 and LAL. In another embodiment, the CHO cell lipase includes LP-PLA2 and LAL. In yet another embodiment, the CHO cell lipase includes PLBL2, LPL, and LPLA2. In still another embodiment, the CHO cell lipase includes PLBL2, LPL, and LP-PLA2. In one embodiment, the CHO cell lipase includes PLBL2, LPL, and LAL. In another embodiment, the CHO cell lipase includes PLBL2, LPLA2, and LP-PLA2. In yet another embodiment, the CHO cell lipase includes PLBL2, LPLA2, and LAL. In still another embodiment, the CHO cell lipase includes PLBL2, LP-PLA2, and LAL. In one embodiment, the CHO cell lipase includes LPL, LPLA2, and LP-PLA2. In another embodiment, the CHO cell lipase includes LPL, LPLA2, and LAL. In yet another embodiment, the CHO cell lipase includes LPL, LP-PLA2, and LAL. In still another embodiment, the CHO cell lipase includes LPLA2, LP-PLA2, and LAL. In one embodiment, the CHO cell lipase includes PLBL2, LPL, LPLA2, and LP-PLA2. In another embodiment, the CHO cell lipase includes PLBL2, LPL, LPLA2, and LAL. In yet another embodiment, the CHO cell lipase includes PLBL2, LPL, LP-PLA2, and LAL. In still another embodiment, the CHO cell lipase includes PLBL2, LPLA2, LP-PLA2, and LAL. In yet still another embodiment, the CHO cell lipase includes PLBL2, LPL, LPLA2 , LP-PLA2, and LAL.

In some embodiments, the production protein is a therapeutic protein. In one embodiment, the production protein is a monoclonal antibody.

In some embodiments of various methods provided herein, the chromatographic resin is an ion exchange (IEX) resin. In other embodiments, the chromatographic resin is a hydrophobic interaction (HIC) resin. In one embodiment, the IEX resin is a cation exchange (CEX) resin. In another embodiment, the CEX resin is a mixed mode CEX resin. In yet another embodiment, the IEX resin is an anion exchange (AEX) resin. In still another embodiment, the AEX resin is a mixed mode AEX resin.

In certain embodiments of various methods using a CEX resin or a mixed mode CEX resin, the pH of the operating condition is below about 6.0. In some embodiments of various methods using a CEX resin or a mixed mode CEX resin, the pH of the operating condition is below about 5.5. In other embodiments of various methods using a CEX resin or a mixed mode CEX resin, the pH of the operating condition is below about 5.0. In yet other embodiments of various methods using a CEX resin or a mixed mode CEX resin, the pH of the operating condition is from about 4.5 to about 5.5. In still other embodiments of various methods using a CEX resin or a mixed mode CEX resin, the pH of the operating condition is from about 4.5 to about 5.0. In certain embodiments of various methods using a CEX resin or a mixed mode CEX resin, the pH of the operating condition is from about 5.0 to about 5.5. In some embodiments of various methods using a CEX resin or a mixed mode CEX resin, the pH of the operating condition is from about 4.9 to about 5.3.

In certain embodiments of various methods using an AEX resin or a mixed mode AEX resin, the pH of the operating condition is above about 6.5. In some embodiments of various methods using an AEX resin or a mixed mode AEX resin, the pH of the operating condition is above about 6.9. In other embodiments of various methods using an AEX resin or a mixed mode AEX resin, the pH of the operating condition is above about 7.2. In yet other embodiments of various methods using an AEX resin or a mixed mode AEX resin, the pH of the operating condition is from about 6.9 to about 7.9. In still other embodiments of various methods using an AEX resin or a mixed mode AEX resin, the pH of the operating condition is from about 7.2 to about 7.5. In certain embodiments of various methods using an AEX resin or a mixed mode AEX resin, the pH of the operating condition is from about 7.5 to about 7.8.

In certain embodiments of various methods provided herein, the operating condition further comprises modulating the ionic strength and/or conductivity of the operating solution by adding a salt. In one embodiment, the operating condition further comprises modulating the ionic strength of the operating solution by adding a salt. In another embodiment, the operating condition further comprises modulating the conductivity of the operating solution by adding a salt. In yet another embodiment, the operating condition further comprises modulating the ionic strength and conductivity of the operating solution by adding a salt. In some embodiments, the effect of adding a salt is to achieve the desired log $\alpha$. In other embodiments, the effect of adding a salt is to achieve the desired log $K_p$ for the lipase. In yet other embodiments, the effect of adding a salt is to achieve the desired log $\alpha$ and the desired log $K_p$ for the lipase.

In some embodiments, the salt in the operating solution is selected from the group consisting of sodium chloride, sodium acetate, sodium phosphate, ammonium sulfate, sodium sulfate, and Tris-HCl. In one embodiment, the salt is sodium chloride. In another embodiment, the salt is sodium acetate. In yet another embodiment, the salt is sodium phosphate. In still another embodiment, the salt is ammonium sulfate. In one embodiment, the salt is sodium sulfate. In another embodiment, the salt is Tris-HCl.

In a specific embodiment, the concentration of sodium chloride in the operating solution is from about 100 mM to about 225 mM, the chromatographic resin is CEX, and the pH of the operating condition is from about 5.0 to about 6.0.

In another specific embodiment, the concentration of sodium chloride in the operating solution is from about 150 mM to about 180 mM, the chromatographic resin is CEX, and the pH of the operating condition is from about 5.0 to about 6.0.

In yet another specific embodiment, the concentration of sodium acetate in the operating solution is from about 100 mM to about 200 mM, the chromatographic resin is AEX; the pH of the operating condition is from about 6.9 to about 7.8.

In still another specific embodiment, the concentration of sodium sulfate in the operating solution is from about 500 mM to about 620 mM, the chromatographic resin is HIC, and the pH of the operating condition is about 7.

In yet still another specific embodiment, the concentration of sodium sulfate in the operating solution is from about 510 mM to about 560 mM, the chromatographic resin is HIC, and the pH of the operating condition is about 7.

In yet another aspect, provided herein is a method of improving PS-80 stability in a production protein formulation, comprising:

    (a) passing a load fluid comprising the production protein through a mixed mode AEX resin;
    (b) collecting the production protein in a flowthrough; and
    (c) formulating the production protein so that the production protein formulation is a PS-80-containing solution;

wherein the pH of the load fluid is from about pH 7.2 to about pH 7.6, and wherein the load fluid does not comprise a salt.

In certain embodiments of such methods, the production protein is a therapeutic protein.

In some embodiments of such methods, the production protein is a monoclonal antibody.

In still another aspect, provided herein is a method of improving PS-80 stability in a production protein formulation, comprising:

    (a) passing a load fluid comprising the production protein through a CEX resin;
    (b) eluting the production protein from the CEX resin with an elution solution; and
    (c) formulating the production protein so that the production protein formulation is a PS-80-containing solution;

wherein the pH of the elution solution is from about pH 4.9 to about pH 5.3, and wherein the elution solution further comprises from about 120 mM to about 175 mM sodium chloride.

In certain embodiments of such methods, the production protein is a therapeutic protein.

In some embodiments of such methods, the production protein is a monoclonal antibody.

In one embodiment, the method of improving PS-80 stability in a production protein formulation comprises:

    (a) passing a load fluid comprising the production protein through a CEX resin;
    (b) eluting the production protein from the CEX resin with an elution solution; and
    (c) formulating the production protein so that the production protein formulation is a PS-80-containing solution;

wherein the pH of the elution solution is about pH 5.1, and wherein the elution solution further comprises about 150 mM sodium chloride.

In certain embodiments of such methods, the production protein is a therapeutic protein.

In some embodiments of such methods, the production protein is a monoclonal antibody.

In yet another embodiment, the method of improving PS-80 stability in a production protein formulation comprises:

    (a) passing a load fluid comprising the production protein through a CEX resin;
    (b) eluting the production protein from the CEX resin with an elution solution; and
    (c) formulating the production protein so that the production protein formulation is a PS-80-containing solution;

wherein the pH of the elution solution is about pH 5.1, and wherein the elution solution further comprises about 165 mM sodium chloride.

In still another aspect, provided herein is a method of improving PS-80 stability in a production protein formulation, comprising:

    (a) passing a load fluid comprising the production protein through a CEX resin;
    (b) eluting the production protein from the CEX resin with an elution solution; and
    (c) formulating the production protein so that the production protein formulation is a PS-80-containing solution;

wherein the pH of the elution solution is from about pH 5.0 to about pH 5.4, and wherein the elution solution further comprises from about 150 mM to about 275 mM sodium chloride.

In one embodiment, the method of improving PS-80 stability in a production protein formulation comprises:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is about pH 5.1, and wherein the elution solution further comprises about 200 mM sodium chloride.

In yet another embodiment, the method of improving PS-80 stability in a production protein formulation comprises:

(a) passing a load fluid comprising the production protein through a CEX resin;
(b) eluting the production protein from the CEX resin with an elution solution; and
(c) formulating the production protein so that the production protein formulation is a PS-80-containing solution;

wherein the pH of the elution solution is about pH 5.1, and wherein the elution solution further comprises about 250 mM sodium chloride.

In certain embodiments of such methods, the production protein is a therapeutic protein.

In some embodiments of such methods, the production protein is a monoclonal antibody.

In more embodiments of the various methods provided herein, the load fluid is an eluate from a prior chromatographic process. In one embodiment, the prior chromatographic process comprises an affinity chromatography. In another embodiment, the prior chromatographic process comprises an affinity chromatography followed by a non-affinity chromatography. In yet another embodiment, the affinity chromatography is a protein A chromatography. In still another embodiment, the non-affinity chromatography is an AEX chromatography. In yet still another embodiment, the prior chromatographic process comprises a protein A chromatography followed by an AEX chromatography.

In another aspect, provided herein is a method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 4.9 to about pH 5.4, and wherein the conductivity of the elution solution is from about 15 mS/cm to about 21 mS/cm.

In certain embodiments of such a method, the production protein is a therapeutic protein.

In other embodiments of such a method, the production protein is a monoclonal antibody.

In another aspect, provided herein is a method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 4.9 to about pH 5.4, and wherein the elution solution further comprises from about 135 mM to about 195 mM sodium chloride.

In certain embodiments of such a method, the production protein is a therapeutic protein.

In other embodiments of such a method, the production protein is a monoclonal antibody.

**8. Pharmaceutical Compositions**

This disclosure also provides pharmaceutical compositions (*e.g.*, drug substance or drug product) comprising a therapeutic protein (*e.g.*, monoclonal antibody) and little amount of a HCP *(e.g.,* lipase).

In certain embodiments, the pharmaceutical composition comprises a therapeutic protein and less than 1 ppm of a host cell lipase. In other embodiments, the pharmaceutical composition comprises a therapeutic protein and less than 0.1, 0.2, 0.3, 0.4, 0.5, 0.6, 0.7, 0.8, or 0.9 ppm of a host cell lipase. In one embodiment, the pharmaceutical composition comprises a therapeutic protein and less than 0.1 ppm of a host cell lipase. In another embodiment, the pharmaceutical composition comprises a therapeutic protein and less than 0.2 ppm of a host cell lipase. In yet another embodiment, the pharmaceutical composition comprises a therapeutic protein and less than 0.3 ppm of a host cell lipase. In still another embodiment, the pharmaceutical composition comprises a therapeutic protein and less than 0.4 ppm of a host cell lipase. In yet still another embodiment, the pharmaceutical composition comprises a therapeutic protein and less than 0.5 ppm of a host cell lipase. In one embodiment, the pharmaceutical composition comprises a therapeutic protein and less than 0.6 ppm of a host cell lipase. In another embodiment, the pharmaceutical composition comprises a therapeutic protein and less than 0.7 ppm of a host cell lipase. In yet another embodiment, the pharmaceutical composition comprises a therapeutic protein and less than 0.8 ppm of a host cell lipase. In still another embodiment, the pharmaceutical composition comprises a therapeutic protein and less than 0.9 ppm of a host cell lipase.

In various embodiments of the pharmaceutical compositions described herein, the level of the host cell lipase is measured by liquid chromatography-mass spectrometry (LC-MS).

In certain embodiments, the pharmaceutical composition is an eluate from a CEX chromatography using an elution solution selected from the group consisting of:

(a) an elution solution with a pH from about 4.9 to about 5.3, comprising from about 120 mM to about 175 mM sodium chloride;

(b) an elution solution with a pH of about 5.1, comprising about 150 mM sodium chloride;

(c) an elution solution with a pH of about 5.1, comprising about 165 mM sodium chloride;

(d) an elution solution with a pH from about 4.9 to about 5.4 and a conductivity from about 15 mS/cm to about 21 mS/cm;

(e) an elution solution with a pH from about 4.9 to about 5.4, comprising from about 135 mM to about 195 mM sodium chloride;

(f) an elution solution with a pH from about pH 5.0 to about pH 5.4, comprising from about 150 mM to about 275 mM sodium chloride;

(g) an elution solution with a pH of about 5.1, comprising about 200 mM sodium chloride; and

(h) an elution solution with a pH of about 5.1, comprising about 250 mM sodium chloride.

In one embodiment, the pharmaceutical composition is an eluate from a CEX chromatography using an elution solution with a pH from about 4.9 to about 5.3, comprising from about 120 mM to about 175 mM sodium chloride.

In another embodiment, the pharmaceutical composition is an eluate from a CEX chromatography using an elution solution with a pH of about 5.1, comprising about 150 mM sodium chloride.

In yet another embodiment, the pharmaceutical composition is an eluate from a CEX chromatography using an elution solution with a pH of about 5.1, comprising about 165 mM sodium chloride.

In still another embodiment, the pharmaceutical composition is an eluate from a CEX chromatography using an elution solution with a pH from about 4.9 to about 5.4 and a conductivity from about 15 mS/cm to about 21 mS/cm.

In one embodiment, the pharmaceutical composition is an eluate from a CEX chromatography using an elution solution with a pH from about 4.9 to about 5.4, comprising from about 135 mM to about 195 mM sodium chloride.

In another embodiment, the pharmaceutical composition is an eluate from a CEX chromatography using an elution solution with a pH from about pH 5.0 to about pH 5.4, comprising from about 150 mM to about 275 mM sodium chloride.

In still another embodiment, the pharmaceutical composition is an eluate from a CEX chromatography using an elution solution with a pH of about 5.1, comprising about 200 mM sodium chloride.

In yet still another embodiment, the pharmaceutical composition is an eluate from a CEX chromatography using an elution solution with a pH of about 5.1, comprising about 250 mM sodium chloride.

In some embodiments of the pharmaceutical compositions, the CEX chromatography is preceded by an AEX chromatography operated in a flowthrough mode.

In certain embodiments of the pharmaceutical compositions, the lipase is selected from the group consisting of PLBL2, LPL, LPLA2, LP-PLA2, and LAL. In one embodiment, the lipase is PLBL2. In another embodiment, the lipase is LPL. In yet another embodiment, the lipase is LPLA2. In one embodiment, the lipase is LP-PLA2. In another embodiment, the lipase is LAL. In still another embodiment, the lipase includes two, three, four, five, six, seven, eight, nine, ten, or more different lipases. In yet still another embodiment, the lipase includes two, three, four, or five different lipases selected from the group consisting of PLBL2, LPL, LPLA2 , LP-PLA2, and LAL. In one embodiment, the lipase includes PLBL2 and LPL. In another embodiment, the lipase includes PLBL2 and LPLA2. In yet another embodiment, the lipase includes PLBL2 and LP-PLA2. In still another embodiment, the lipase includes PLBL2 and LAL. In one embodiment, the lipase includes LPL and LPLA2. In another embodiment, the lipase includes LPL and LP-PLA2. In yet another embodiment, the lipase includes LPL and LAL. In still another embodiment, the lipase includes LPLA2 and LP-PLA2. In one embodiment, the lipase includes LPLA2 and LAL. In another embodiment, the lipase includes LP-PLA2 and LAL. In yet another embodiment, the lipase includes PLBL2, LPL, and LPLA2. In still another embodiment, the lipase includes PLBL2, LPL, and LP-PLA2. In one embodiment, the lipase includes PLBL2, LPL, and LAL. In another embodiment, the lipase includes PLBL2, LPLA2, and LP-PLA2. In yet another embodiment, the lipase includes PLBL2, LPLA2, and LAL. In still another embodiment, the lipase includes PLBL2, LP-PLA2, and LAL. In one embodiment, the lipase includes LPL, LPLA2, and LP-PLA2. In another embodiment, the lipase includes LPL, LPLA2, and LAL. In yet another embodiment, the lipase includes LPL, LP-PLA2, and LAL. In still another embodiment, the lipase includes LPLA2, LP-PLA2, and LAL. In one embodiment, the lipase includes PLBL2, LPL, LPLA2, and LP-PLA2. In another embodiment, the lipase includes PLBL2, LPL, LPLA2, and LAL. In yet another embodiment, the lipase includes PLBL2, LPL, LP-PLA2, and LAL. In still another embodiment, the lipase includes PLBL2, LPLA2, LP-PLA2, and LAL. In yet still another embodiment, the lipase includes PLBL2, LPL, LPLA2 , LP-PLA2, and LAL.

In other embodiments of the pharmaceutical compositions, the therapeutic protein is a monoclonal antibody.

## VI. EXAMPLES

The examples in this section (section VI) are offered by way of illustration, and not by way of limitation.

### Example 1: Method for determining $K_P$ of different species

A partitioning coefficient, $K_P$, is determined by mixing a known liquid concentration of protein (or other molecule of interest) with a known volume of chromatography resin and calculating the ratio of the protein bound to the resin and the protein remaining in the liquid:

$$K_P = q/c = [bound]/[free].$$

For all subsequent examples, the chromatography volume was 20 $\mu$L, and the liquid volume was 200 $\mu$L with a protein concentration of 0.5 mg/mL. These volumes provide a phase ratio of 10: 1 for an effective resin loading of 5 mg/mL.

Screenings were conducted by vigorous mixing of resin and liquid in a 96-well filter plate (PIN MSBVN1250, Millipore Sigma, Burlington, MA) with separation of resin and liquid by vacuum filtration. The sequence of steps was as follows:

(a) 3 x equilibration (buffer not containing feed), 10 min incubation each step;
(b) 1 x feed mixing, 60 min incubation; and
(c) 2 x strip conditions, 10 min incubation each step

The equilibration step allows for buffer exchange from the initial resin slurry buffer. The 60 min time for feed mixing allows for pseudo equilibration between the resin ligand and protein at a given set of conditions. The filtrate from the feed step was measured by UV absorbance at 280 - 320 nm to determine the final liquid concentration of the protein, c. The bound concentration of the protein, q, was determined by a mass balance around c and the known feed concentration, $c_0$ (0.5 mg/mL).

Partitioning is generally reported in terms of log $K_P$, which can be accurately quantified from approximately 0 to 2 using the UV method described here. General rules for log $K_P$ screening are as follows:

$$\log K_P \geq 1.5,$$

strong binding to the resin;

$$\log K_P < 1,$$

conditions where elution would be expected for a bind-and-elute modality;

$$0.5 < \log K_P < 1,$$

weak interaction conditions that will show some binding;

$$\log K_P < 0.5,$$

very little or no binding.

The log Kp of different species is also used to predict separation of different species through the calculation of a separation factor, $\alpha$, as follows: $\alpha = K_{P, protein\,1}/K_{P, protein\,2}$; $\log \alpha = \log K_{P, protein\,1} - \log K_{P, protein\,2}$, where a log $\alpha$ further from 0

indicates better separation. In the following examples, $\alpha = K_{P,\,lipase}/K_{P,\,mAb}$; $\log \alpha = \log K_{P,\,lipase} - \log K_{P,\,mAb}$. A $\log \alpha$ larger than 0.5 indicates good separation between the lipase and a monoclonal antibody.

**Example 2: Comparison of PLBL2 and mAb $K_P$ values at typical processing conditions**

The method for determining $K_P$ and $\alpha$ was used to assess the capability of separating a known lipase impurity, PLBL2, at operating conditions for two monoclonal antibodies, mAb1 and mAb2, through a variety of chromatographic processes. **Table 1** summarizes the log Kp and log $\alpha$ values for mAb1 and PLBL2 at several process conditions for mAb1.

**Table 1. log Kp and log $\alpha$ values for mAb1 and PLBL2 at processing conditions for mAb1**

| Process, Resin | Operating Condition | mAb1 log $K_P$ | PLBL2 log $K_P$ | log $\alpha$ |
|---|---|---|---|---|
| Protein A, Mab-Select Sure (GE Healthcare, Chicago, IL) | Equil/wash: 10 mM NaPhoshpate, pH 6.5 | 2 | 0 | -2 |
| | High salt wash: 10 mM NaPhosphate, pH 6.5, 0.5 M NaCl | 2 | 0 | -2 |
| | Elute: 20 mM NaAcetate, pH 3.5 | 0 | 0 | 0 |
| | Strip: 100 mM acetic acid | 0 | 0 | 0 |
| AEX, Poros HQ 50 (Thermo Fisher Scientific, Waltham, MA) | Load: 100 mM NaAcetate, 100 mM Tris, pH 7.5 | 0.3 | 1.6 | 1.3 |
| | Wash: 25 mM NaPhosphate, pH 7.2 | 0 | 1.4 | 1.4 |
| | Strip: 1 M NaCl | 0 | 0 | 0 |
| CEX, Poros HS 50 (Thermo Fisher Scientific, Waltham, MA) | Load: 100 mM NaAcetate, 100 mM Tris, pH 5.1 | 2 | 2 | 0 |
| | Elute (low salt limit): 20 mM NaAcetate, pH 5.1, 125 mM NaCl | 1.5 | 1.8 | 0.3 |
| | Elute (center point): 20 mM NaAcetate, pH 5.1, 150 mM NaCl | 0.5 | 1.6 | 1.1 |
| | Elute (high salt limit): 20 mM NaAcetate, pH 5.1, 175 mM NaCl | 0 | 1.4 | 1.4 |
| | Strip: 1 M NaCl | 0 | 0 | 0 |

**Table 1** shows potential operating conditions for separating PLBL2 from mAB1 through a chromatographic process.

For the protein A process, PLBL2 has no affinity, so the majority of PLBL2 would be expected to flow through the protein A resin during loading or wash steps. The only PLBL2 present in pools would likely be from insufficient washes or associated with mAb1.

For the AEX process, which is operated in flowthrough mode for mAb1, PLBL2 shows stronger binding (higher log $K_P$) compared to mAb1 at loading and wash conditions. This results in a log $\alpha$ larger than 1.0 at these conditions, which indicates that PLBL2 would remain bound to the resin at these conditions, whereas mAb1 would flow through.

For the CEX process, PLBL2 shows less sensitivity to salt modulation and has a higher log $K_P$ at the higher ends of the salt range, compared to mAb1. A log $\alpha$ larger than 1.0 at the center point and high salt limit indicates that PLBL2 would remain bound during mAb1 elution. The low salt limit provides a much less favorable log $\alpha$ for separation of PLBL2 from mAb1, which is expected to retain some binding at these conditions (log $K_{P,\,mAb1}$ of 1.5).

**Table 2** summarizes the log Kp and log $\alpha$ values for mAb2 and PLBL2 at several process conditions for mAb2.

**Table 2. log $K_P$ and log $\alpha$ values for mAb2 and PLBL2 at processing conditions for mAb2**

| Process, Resin | Operating Condition | mAb2 log $K_P$ | PLBL2 log $K_P$ | log $\alpha$ |
|---|---|---|---|---|
| Protein A, MabSelect Sure | Equil/wash: 10 mM NaPhoshpate, pH 6.5 | 2 | 0 | -2 |
| | High salt wash: 10 mM NaPhosphate, pH 6.5, 0.5 M NaCl | 2 | 0 | -2 |
| | Elute: 20 mM NaAcetate, pH 3.5 | 0.3 | 0 | -0.3 |
| | Strip: 100 mM acetic acid | 0 | 0 | 0 |

(continued)

| Process, Resin | Operating Condition | mAb2 log $K_P$ | PLBL2 log $K_P$ | log $\alpha$ |
|---|---|---|---|---|
| CEX, Poros HS 50 | Load: 100 mM NaAcetate, 100 mM Tris, pH 5.1 | 2 | 2 | 0 |
| | Elute (low salt limit): 20 mM NaAcetate, pH 5.1, 125 mM NaCl | 0.2 | 1.8 | 1.6 |
| | Elute (center point): 20 mM NaAcetate, pH 5.1, 150 mM NaCl | 0 | 1.6 | 1.6 |
| | Elute (high salt limit): 20 mM NaAcetate, pH 5.1, 175 mM NaCl | 0 | 1.4 | 1.4 |
| | Strip: 1 M NaCl | 0 | 0 | 0 |

**Table 2** shows potential operating conditions for separating PLBL2 from mAB2 through a chromatographic process.

The trends of mAb2 for the protein A and CEX processes are very similar to that of mAb1, with mAb2 demonstrating slightly stronger binding during the protein A elution and weaker binding during the CEX elution. For the CEX process, mAb2 has lower binding at lower salt and therefore a more robust log $\alpha$ throughout the salt range.

### Example 3: Mapping of PLBL2 and LPLA2 $K_P$ values at a range of conditions for different resins

An extensive mapping was performed to determine the partitioning coefficient of PLBL2 and LPLA2 for a wide variety of resins with different buffers and conditions that might potentially be used in downstream processing (**Table 3**). Salt and pH conditions were tested combinatorially. The comprehensive mapping of PLBL2 and LPLA2 $K_p$ can provide a basis for predicting separation of PLBL2 or LPLA2 with known mAb purification conditions or for conditions to be explored.

### Table 3. Conditions screened for mapping PLBL2 or LPLA2 log $K_p$

| Modality | Buffer | Salt | Salt Concentration (mM) | pH |
|---|---|---|---|---|
| AEX | Tris-acetate | sodium acetate | 50, 100, 150 | 7.0, 7.2, 7.5 |
| | Tris | sodium chloride | 0, 50, 100, 150 | 6.9, 7.2, 7.5, 7.8 |
| | Phosphate | sodium chloride | 0, 25, 50 | 6.9, 7.2 |
| CEX - salt screen | Acetate | sodium chloride | 100, 125, 150, 175, 200, 225, 250, 275, 300 | 5.0, 5.2, 5.4, 5.6 |
| CEX - pH screen | Acetate or phosphate | sodium chloride | 0, 25, 50 | 5.0, 5.5, 6.0, 6.4, 6.8, 7.2, 7.6 |
| HIC | Phosphate | sodium sulfate | 0, 100, 150, 200, 250, 300, 350, 400, 450, 500, 550, 600 | 7.0 |
| Mixed mode AEX | Tris | sodium chloride | 0, 25, 50, 100, 150, 200 | 7.2, 7.5, 7.8 |
| Mixed mode CEX | Acetate | sodium chloride | 0, 100, 150, 200, 250, 300, 400, 500 | 5.0, 5.5, 6.0 |

3.1 AEX chromatography

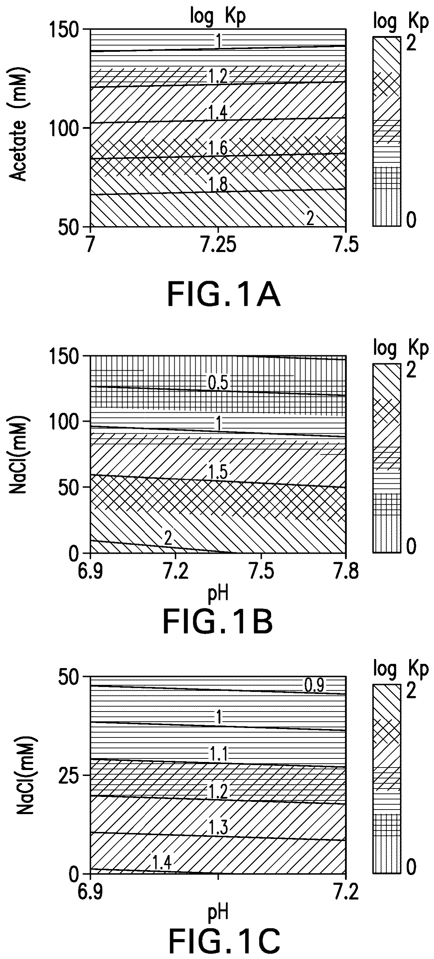

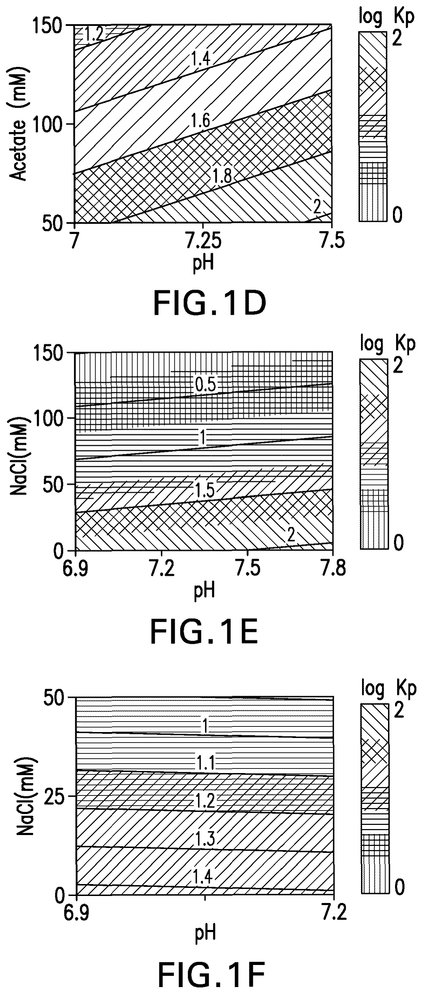

A set of conditions for AEX chromatography is listed in Table 3 and depicted in **FIGS. 1A-1C** for PLBL2 and in **FIGS. 1D-1F** for LPLA2 with Poros 50 HQ resin. The first buffer combination represents a mixture of buffers in ranges that might commonly be seen for AEX loading steps in flowthrough mode following protein A and low pH hold steps (**FIGS. 1A and 1D**). Under these conditions, acetate acted as the counter ion to compete for binding, and Tris base was added in appropriate amounts to control pH. PLBL2 showed strong interactions even at > 100 mM acetate additions, with a log $K_P$ of approximately 1 observed at about 140 mM acetate with log $K_p$ presumably continuing to drop at higher acetate concentrations (**FIG. 1A**). Little difference was observed across the pH 7.0-7.5 range (**FIG. 1A**). LPLA2 showed similar trends with acetate but also showed a stronger dependence on pH with stronger binding seen at higher pH (**FIG. 1D**).

Two other AEX conditions represent buffers (Tris and Phosphate) that might be used for AEX equilibration and wash steps. The use of NaCl salt modulation provides possible conditions for AEX process following a prior salt elution (e.g., from CEX). In Tris buffer (**FIGS. 1B and 1E**), PLBL2 remains strongly bound (log $K_P$ > 1.5) up to about 50 mM NaCl addition, and log $K_P$ drops below 1 above approximately 100 mM NaCl. LPLA2 behaves similar but is less strongly retained with log Kp > 1.5 up to about 30 mM NaCl and log Kp < 1 above about 75 mM NaCl. The phosphate buffer (**FIGS. 1C and 1F**) prevents PLBL2 and LPLA2 interactions more strongly than Tris; no conditions screened with either lipase provided log $K_P$ > 1.5, and log $K_P$ dropped below 1 at about 40 mM NaCl in each case. pH had little effect for either buffer across the ranges tested.

### 3.2 CEX chromatography

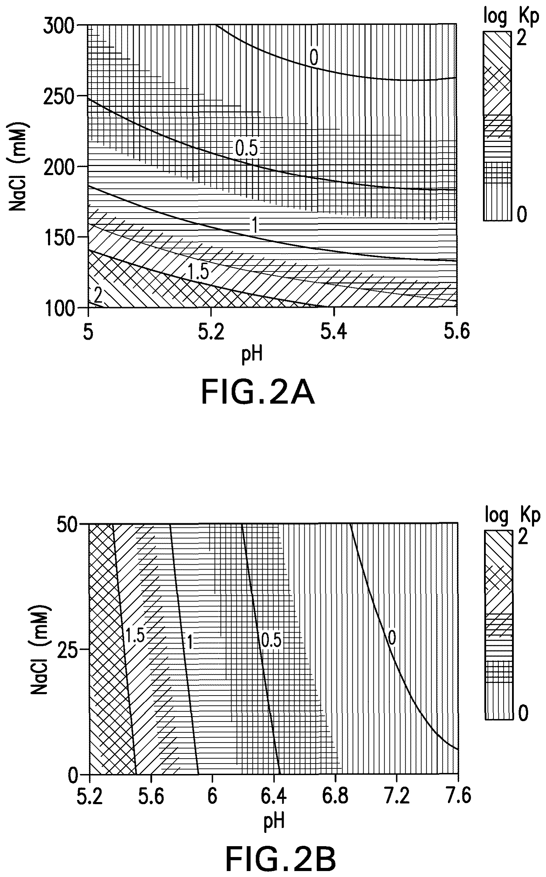

A set of conditions for CEX chromatography is listed in **Table 3** and depicted in **FIGS. 2A-2C** for Poros 50 HS resin.

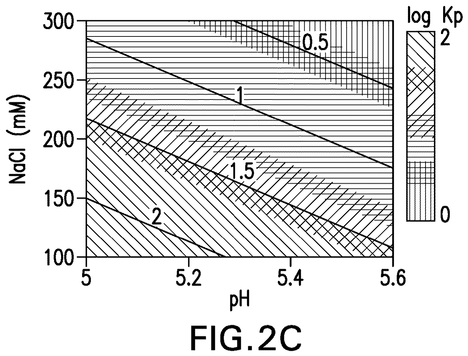

The first combination represents a pH and salt range that might typically be used for binding and elution of mAbs using NaCl modulation for elution (**FIGS. 2A and 2C**). In this case, NaCl had a strong effect with no binding seen above 250 mM NaCl and log $K_P$ values of 1 around 150 mM NaCl for PLBL2 (**FIG. 2A**). pH also had a significant impact with increasing log $K_p$ at lower pH, particularly closer to pH 5.0. LPLA2 demonstrated similar trends for pH and salt modulation but had significantly stronger retention with log $K_p$ values of 1 between 200-250 mM NaCl (**FIG. 2C**).

The second combination represents conditions where pH is used to modulate binding, typically at much lower salt conditions (**FIG. 2B**). In this case, strong PLBL2 binding above log $K_P$ of 1.5 was only seen below pH 5.5, and log $K_P$ values drop below 1 above about pH 5.8. The salt conditions tested here had little impact on partitioning at these pH values. Acetate buffer was used up to pH 6.0, and phosphate buffer was used to buffer higher pH conditions.

### 3.3 HIC

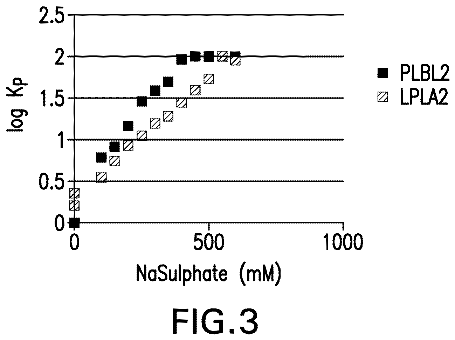

Testing for partitioning of PLBL2 and LPLA2 to a HIC resin, Tosoh Butyl-650M, was conducted by modulating sodium sulfate concentration at a buffering condition of 20 mM sodium phosphate (pH 7.0) (**Table 3, FIG. 3**). Both lipases showed typical HIC behavior with strong binding at high salt (log $K_P$ > 1.5 above 250 mM sodium sulfate for PLBL2 and 400 mM sodium sulfate for LPLA2) and decreased partitioning at lower salt (log $K_P$ < 1 below 150 mM sodium sulfate for PLBL2 and 200 mM sodium sulfate for LPLA2).

### 3.4 Multimodal chromatography

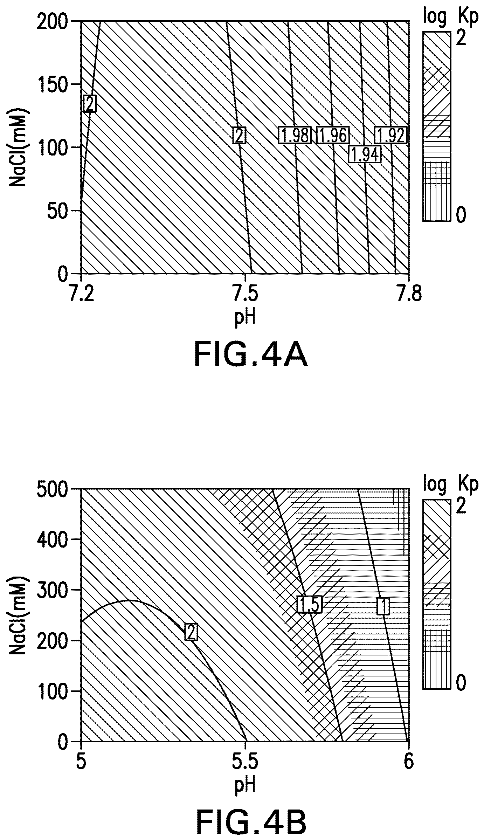

Partitioning of PLBL2 was also tested on two multimodal chromatography resins: a multimodal AEX resin, Capto adhere, and a multimodal CEX resin, Capto MMC (**Table 3**, **FIGS. 4A and 4B**). For Capto adhere, log $K_P$ values were greater than 1.9 at all conditions tested, demonstrating strong binding over a wide range of operating conditions (**FIG. 4A**). For Capto MMC, binding was predominately modulated by pH changes across a much wider salt range (**FIG. 4B**). Strong binding range with log $K_P$ above 1.5 was observed below about pH 5.8. Weaker binding range with log $K_P$ less than 1 was only observed above pH of approximately 5.9 with high salt additions.

### Example 4: Optimization of conditions to separate PLBL2 from an IgG1 mAb, mAb3

The partitioning maps provided for PLBL2 in **Example 3** were used to optimize separation of PLBL2 from an IgG1 mAb, mAb3. The performance of mAb3 was similar to that of mAb1 for the protein A process. Strong binding of mAb3 was observed under protein A loading conditions (data not shown).

### 4.1 AEX chromatography

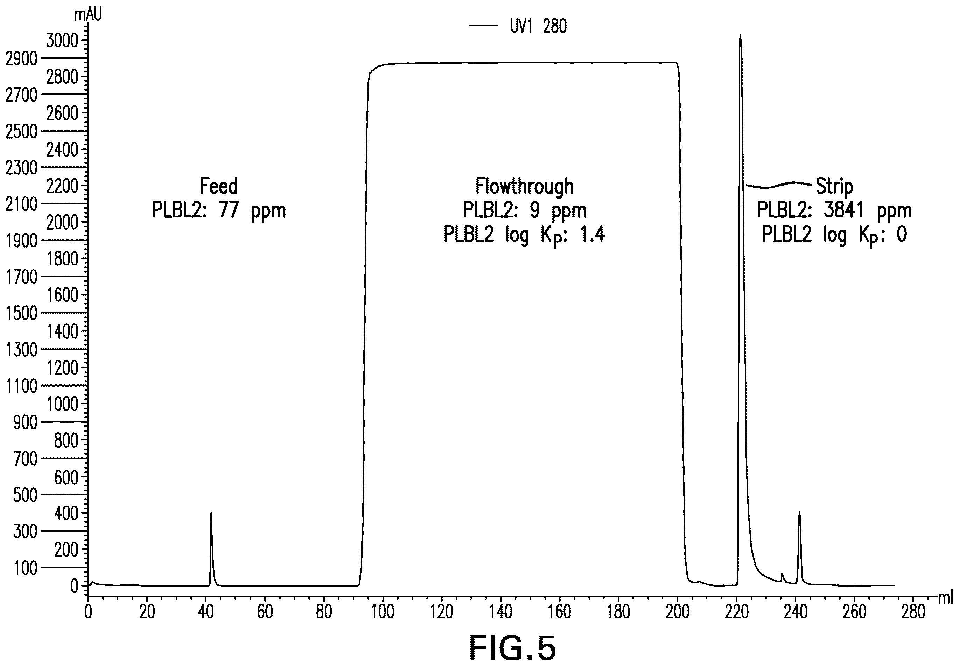

The column used was approximately 7 mL volume at a 20 cm bed height for Poros HQ resin. The mAb3 feed was at 13.5 mg/mL concentration in a Tris and acetate mixture at pH 7.5 with the acetate counterion at approximately 110 mM. The mAb3 log $K_P$ at this condition is close to zero whereas the PLBL2 log $K_P$ is around 1.4 (**FIG. 1A**), indicating that mAb3 will not but PLBL2 will bind to the resin to certain extent. The process was run in flowthrough mode, and the chromatogram indicates that very little mAb3 bound to the column (**FIG. 5**). PLBL2 in different fractions was quantified using a mass spectrometry method on a QE HF-X system with known PLBL2 peptides used to calibrate concentrations. The concentration of PLBL2 in the feed was 77 ppm. The concentration of PLBL2 in the flowthrough was 9 ppm. The concentration of PLBL2 in the strip was 3841 ppm. The detected amounts indicate that over 85% of PLBL2 was removed from mAb3 in the flowthrough pool. High amounts of PLBL2 in the strip pool indicate that the lipase bound to the resin under the flowthrough condition, as predicted by the log $K_P$ value of 1.4, and was eluted under the 1 M NaCl high salt strip condition, also predicted by the log $K_P$ value of 0.

Thus, protein A and AEX represent promising steps to remove PLBL2 at common operating conditions with log $\alpha$ values of -2 during protein A loading and of approximately 1.5 during AEX loading at flowthrough mode. Similar $\alpha$ values can be achieved with LPLA2 for ProA and even better separation up to approximately 1.7 for AEX loading in flowthrough mode.

For mAb3, additional clearance beyond the protein A and AEX processes was also desired, so log $K_P$ maps were generated at similar conditions to those used for PLBL2 and LPLA2 in **Example 3.** These log $K_P$ values were then used to calculate log $\alpha$ across the ranges screened to identify the conditions of greatest separation.

<u>4.2 CEX chromatography</u>

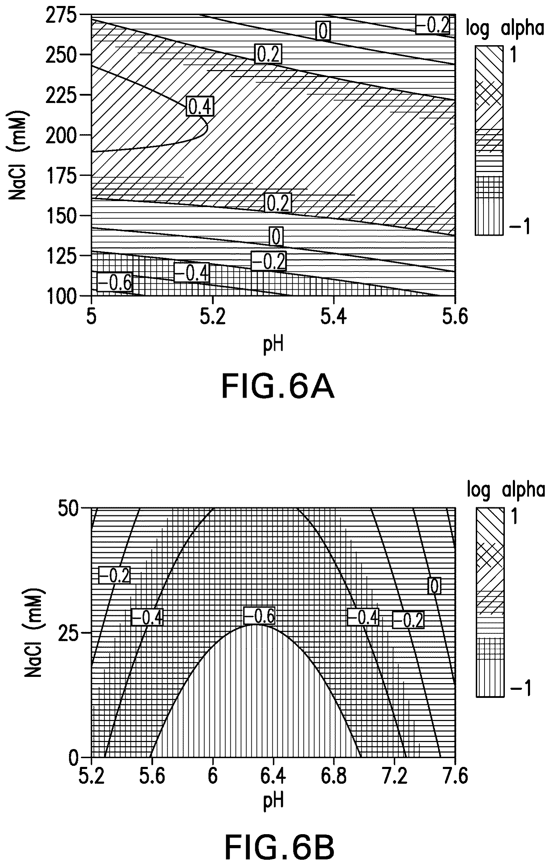

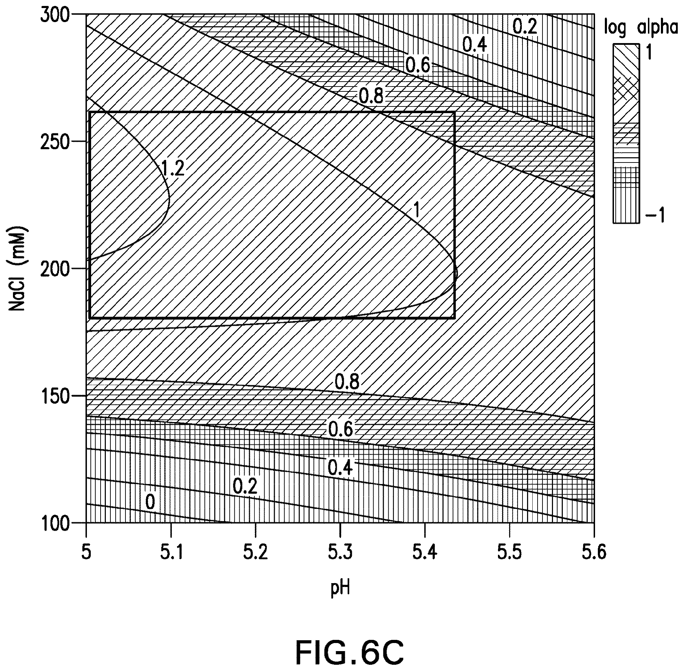

Conditions for separation using CEX chromatography with Poros 50 HS resin are depicted as log $\alpha$ values in **FIGS. 6A and 6B** for PLBL2 and in **FIG. 6C** for LPLA2 at the conditions listed in **Table 3.** For modulating binding with salt, the best separation conditions for PLBL2 were between pH 5-5.2 and 200-225 mM NaCl where log $\alpha$ values are approximately 0.4 (**FIG. 6A,** black box). Since the log $\alpha$ is positive, mAb3 is bound less strongly than PLBL2 indicating that, under these conditions, mAb3 would elute form the resin while PLBL2 might remain bound. The optimized area represents a somewhat narrow pH and salt range and does not have a particularly high log $\alpha$. Confirmation using traditional column chromatography would still likely need to be performed for this process.

The separation for LPLA2 and mAb3 over these CEX conditions is much greater as shown in **FIG. 6C.** While the optimized range is similar to PLBL2, the $\alpha$ value for LPLA2 and mAb3 is greater than 1 at conditions from 200-250 mM NaCl and pH of 5.0-5.3.

In contrast to salt modulation, changing binding by pH produced negative log $\alpha$ values of approximately -0.6 (**FIGS. 6B**, black box). The region encompassing approximately pH 6.0-6.6 and less than 20 mM NaCl represents conditions where mAb3 bound more strongly to the resin than PLBL2. Thus, these conditions might be used as an intermediate wash where PLBL2 could be removed before eluting mAb3 at a higher pH (and/or higher salt).

To evaluate whether an additional CEX process can further separate PLBL2 and LPLA2 from mAb3 after an AEX process operated in flowthrough mode under the loading operating condition specified in **Example 4.1,** an AEX flowthrough pool was loaded on a CEX column containing Poros HS resin, operated in bind-and-elute mode with elution condition at pH 5.1 and 165 mM NaCl. The log $\alpha$ under this elution operating condition is 0.2 for PLBL2 (**FIG. 6A**) and 0.9 for LPLA2 (**FIG. 6C**). While these log $\alpha$ values are less than the value in the optimum range around 200 mM NaCl, the positive values still indicate that lipases will bind more strongly to the resin under this elution condition, particularly for LPLA2. Mass spectrometry analysis showed that the PLBL2 in the CEX feed was 5 ppm but the PLBL2 in the CEX elution pool dropped to 0.3 ppm. LPLA2 in the elution pool was below detection limits (data not shown). These results demonstrate that, under the elution operating condition specified, an additional CEX process can further separate PLBL2 from mAb3 after the AEX process run in flowthrough mode.

<u>4.3 HIC</u>

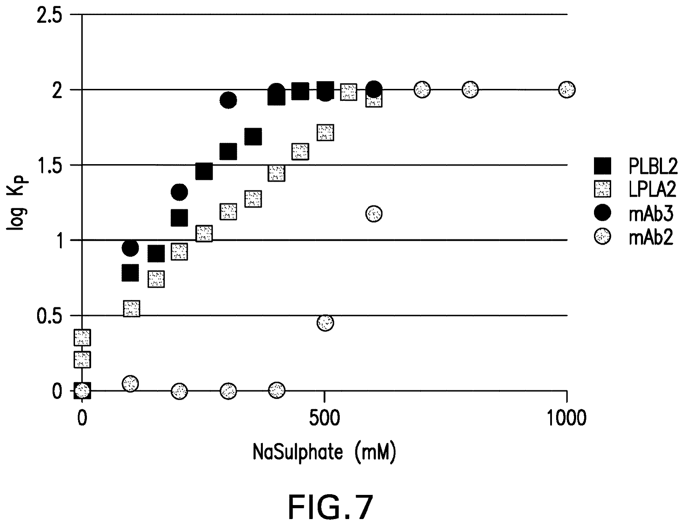

Partitioning of mAb3 and lipases was also compared on a HIC resin, Tosoh Butyl-650M, (**FIG. 7**) at the conditions listed in **Table 3.** Varying sodium sulfate concentration provides little separation between the mAb3 and PLBL2 with only 300 mM sodium sulfate providing any separation at all with a log $\alpha$ of approximately 0.3 at this condition. LPLA2 provides somewhat better separation with log $\alpha$ of about 0.5 between 300-400 mM sodium sulfate. In contrast, mAb2 is much less hydrophobic than mAb3, PLBL2, or LPLA2, and thus does not transition to strong binding to the HIC resin above log $K_P$ of 1.5 until greater than 600 mM sodium sulfate. For mAb2 and PLBL2, log $\alpha$ values from 1.5-2.0 can be achieved between 300-500 mM sodium sulfate, a very wide salt range with promising separation capabilities for operating within. Similarly for LPLA2, log $\alpha$ values greater than 1 are seen in this same salt range.

<u>4.4 Multimodal chromatography</u>

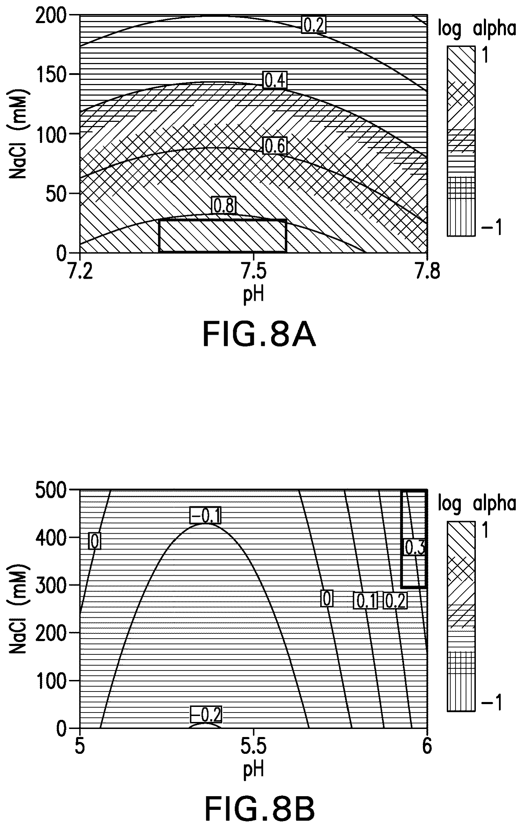

Partitioning of mAb3 and PLBL2 was finally compared for different multimodal resins, multimodal AEX resin, Capto adhere (**FIG. 8A**) and multimodal CEX resin, Capto MMC (**FIG. 8B**) at the conditions listed in **Table 3.**

For Capto adhere, PLBL2 bound strongly at all conditions (**FIG. 4A**), whereas mAb3 demonstrated hydrophobic interactions with stronger partitioning at higher salt. The resulting log $\alpha$ plot shows the highest separation factor (log $\alpha$ > 0.8) under conditions of pH 7.3-7.6 at low salt where mAb3 binding was lowest (**FIG. 8A**). These conditions could be utilized for mAb3 elution or flowthrough while maintaining PLBL2 binding to the Capto adhere resin.

Capto MMC does not provide the same level of separation of mAb3 and PLBL2 as Capto adhere for the conditions screened. The best conditions were seen at pH 5.9-6.0 and above 300 mM NaCl, where log $\alpha$ values up to 0.3 were observed. Because the log $\alpha$ is greater than zero, these conditions might be used for mAb3 elution while maintaining PLBL2 binding (**FIG. 8B**). A wider or more favorable set of separation conditions might be possible at higher pH and/or higher salt because the optimal range was seen at the upper limits of both factors in this screen.

**Example 5: Optimization of CEX operating conditions for a monoclonal antibody mAb4**

Five factors were studied in the CEX Design of Experiment (DoE) as displayed in **Table 4.** Elution buffer pH and conductivity were adjusted using variable amounts of sodium acetate trihydrate, 4 M acetic acid, and sodium chloride. Load pH and conductivity were adjusted using 1 M Tris, 1 M acetic acid, and 1 M sodium chloride.

**Table 4. CEX DoE factors to be studied**

| Factor | Units | Design Levels | | | | |
|---|---|---|---|---|---|---|
| | | Axial Low | Low | Middle | High | Axial High |
| **Elution pH** | - | 4.8 | 4.9 | 5.1 | 5.3 | 5.4 |
| **Elution Conductivity** | mS/cm | 15 | 16 | 18 | 20 | 21 |
| **Load pH** | - | N/A | 4.9 | 5.1 | 5.3 | N/A |
| **Load Conductivity** | mS/cm | N/A | 4 | 6 | 8 | N/A |
| **Resin Loading** | g/L | 20 | 30 | 40 | 50 | 60 |

The DoE design came to a total of 40 runs as displayed in **Table 5.** Each elution buffer was prepared twice with the center-point elution buffer prepared three times.

**Table 5. CEX DoE runs**

| Run (Randomized) | Standard | Type | Elution Buffer Preparations (Whole Plots) | Elution buffer pH | Elution buffer conductivity (mS/cm) | Load pH | Load conductivity (mS/cm) | Resin load (g/L) |
|---|---|---|---|---|---|---|---|---|
| 1 | 32 | Overall Center | 1 | 5.1 | 18 | 5.1 | 6 | 40 |
| 2 | 31 | Overall Center | 1 | 5.1 | 18 | 5.1 | 6 | 40 |
| 3 | 10 | Factorial | 2 | 5.3 | 16 | 4.9 | 8 | 31 |
| 4 | 21 | Axial | 3 | 5.1 | 15 | 5.1 | 6 | 40 |
| 5 | 11 | Factorial | 4 | 5.3 | 16 | 5.3 | 4 | 31 |
| 6 | 16 | Factorial | 5 | 5.3 | 20 | 5.3 | 8 | 31 |
| 7 | 34 | Overall Center | 6 | 5.1 | 18 | 5.1 | 6 | 40 |
| 8 | 38 | Ext. Factorial | 7 | 4.8 | 21 | 5.1 | 6 | 40 |
| 9 | 7 | Factorial | 8 | 4.9 | 20 | 5.3 | 4 | 31 |
| 10 | 20 | Axial | 9 | 5.4 | 18 | 5.1 | 6 | 40 |
| 11 | 15 | Factorial | 10 | 5.3 | 20 | 5.3 | 4 | 50 |
| 12 | 9 | Factorial | 4 | 5.3 | 16 | 4.9 | 4 | 50 |
| 13 | 19 | Axial | 11 | 5.4 | 18 | 5.1 | 6 | 40 |
| 14 | 33 | Overall Center | 6 | 5.1 | 18 | 5.1 | 6 | 40 |
| 15 | 12 | Factorial | 2 | 5.3 | 16 | 5.3 | 8 | 50 |
| 16 | 29 | Axial | 12 | 5.1 | 18 | 5.1 | 6 | 20 |
| 17 | 18 | Axial | 13 | 4.8 | 18 | 5.1 | 6 | 40 |
| 18 | 5 | Factorial | 8 | 4.9 | 20 | 4.9 | 4 | 50 |

(continued)

| Run (Randomized) | Standard | Type | Elution Buffer Preparations (Whole Plots) | Elution buffer pH | Elution buffer conductivity (mS/cm) | Load pH | Load conductivity (mS/cm) | Resin load (g/L) |
|---|---|---|---|---|---|---|---|---|
| 19 | 39 | Ext. Factorial | 14 | 5.4 | 15 | 5.1 | 6 | 40 |
| 20 | 40 | Ext. Factorial | 15 | 5.4 | 15 | 5.1 | 6 | 40 |
| 21 | 8 | Factorial | 16 | 4.9 | 20 | 5.3 | 8 | 50 |
| 22 | 4 | Factorial | 17 | 4.9 | 16 | 5.3 | 8 | 31 |
| 23 | 1 | Factorial | 18 | 4.9 | 16 | 4.9 | 4 | 31 |
| 24 | 13 | Factorial | 5 | 5.3 | 20 | 4.9 | 4 | 31 |
| 25 | 23 | Axial | 19 | 5.1 | 21 | 5.1 | 6 | 40 |
| 26 | 37 | Ext. Factorial | 20 | 4.8 | 21 | 5.1 | 6 | 40 |
| 27 | 3 | Factorial | 18 | 4.9 | 16 | 5.3 | 4 | 50 |
| 28 | 27 | Axial | 12 | 5.1 | 18 | 5.1 | 4 | 40 |
| 29 | 17 | Axial | 21 | 4.8 | 18 | 5.1 | 6 | 40 |
| 30 | 30 | Axial | 12 | 5.1 | 18 | 5.1 | 6 | 60 |
| 31 | 6 | Factorial | 16 | 4.9 | 20 | 4.9 | 8 | 30 |
| 32 | 26 | Axial | 12 | 5.1 | 18 | 5.3 | 6 | 40 |
| 33 | 22 | Axial | 22 | 5.1 | 15 | 5.1 | 6 | 40 |
| 34 | 25 | Axial | 12 | 5.1 | 18 | 4.9 | 6 | 40 |
| 35 | 14 | Factorial | 10 | 5.3 | 20 | 4.9 | 8 | 50 |
| 36 | 24 | Axial | 23 | 5.1 | 21 | 5.1 | 6 | 40 |
| 37 | 28 | Axial | 12 | 5.1 | 18 | 5.1 | 8 | 40 |
| 38 | 2 | Factorial | 17 | 4.9 | 16 | 4.9 | 8 | 50 |
| 39 | 35 | Overall Center | 24 | 5.1 | 18 | 5.1 | 6 | 40 |
| 40 | 36 | Overall Center | 24 | 5.1 | 18 | 5.1 | 6 | 40 |

Each run was operated according to the steps in **Table 6** on a 7 mL chromatography column. The elution pools and strips of each run were collected for yield and quality analysis.

**Table 6. CEX operating steps**

| Step | Buffer | Target pH | Target Conductivity (mS/cm) | Flow Rate (cm/hr) | Column Volume | Flow |
|---|---|---|---|---|---|---|
| **Sanitization** | 0.5 M NaOH | > 12 | 83 - 105 | 300 | ≥ 3 | Down |
| **Equilibration 1** | 1 M NaCl | NA | 75 - 95 | 300 | ≥ 3 | Down |
| **Equilibration 2** | 20 mM Sodium Acetate | 4.9 - 5.3 (DoE) | ≤ 2.0 | 300 | ≥ 5 | Down |

(continued)

| Step | Buffer | Target pH | Target Conductivity (mS/cm) | Flow Rate (cm/hr) | Column Volume | Flow |
|---|---|---|---|---|---|---|
| **Load** | CEX Co-lumm Load | 4.9 - 5.3 (DoE) | 4 - 8 (DoE) | 300 | ~5 | Down |
| **Wash** | 20 mM Sodium Acetate | 4.9-5.3 (DoE) | ≤ 2.0 | 300 | ≥ 3 | Down |
| **Elution** | 20 mM Sodium Acetate, 135-195 mM NaCl (DoE) | 4.8-5.4 (DoE) | 16-21 (DoE) | 300 | ≥0.250 AU / cm ≤1.5 AU / cm Total Approximate CV of 2-6 | Down |
| **Strip** | 1 M NaCl | NA | 75 - 95 | 300 | ≥ 3 | Down |
| **Regeneration** | 0.5 M NaOH | > 12 | 83 - 105 | 300 | ≥ 3 | Down |
| **Storage** | 0.1 M NaOH | > 12 | 18 - 24 | 300 | ≥ 3 | Down |

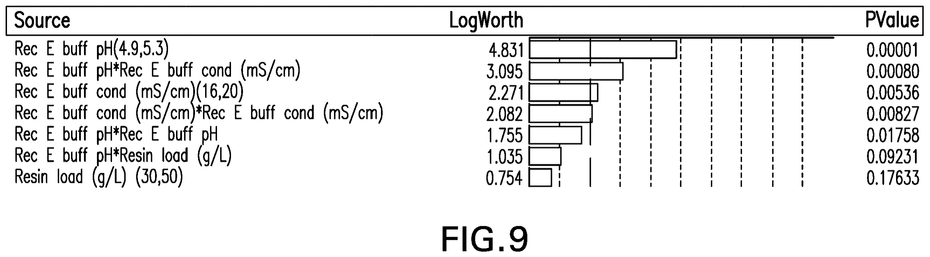

The elution pools and strips of each run were submitted for residual HCP ELISA analysis. Elution buffer pH and conductivity were found to have the greatest effect on residual HCP ELISA results (**FIG. 9**).

Hence, specific runs based on elution buffer conditions were analyzed further by liquid chromatography-mass spectrometry (LC-MS) (see **Table 7**). Due to material limitations, the pools and strips of some runs with the same elution buffer conditions were combined (*e.g.*, 10&13 below).

**Table 7. CEX DoE pools and strips submitted for LCMS analysis**

| Run # | Elution Buffer pH | Elution Buffer Conductivity (mS/cm) | Load pH | Load Conductivity (mS/cm) | Resin Loading (g/L) |
|---|---|---|---|---|---|
| 10&13 | 5.4 | 18 | 5.1 | 6 | 40 |
| 19&20 | 5.4 | 15 | 5.1 | 6 | 40 |
| 11&24 | 5.3 | 20 | 5.1* | 4* | 40* |
| 12 | 5.3 | 16 | 4.9 | 4 | 50 |
| 25 | 5.1 | 21 | 5.1 | 6 | 40 |
| 1&2 | 5.1 | 18 | 5.1 | 6 | 40 |
| 34 | 5.1 | 18 | 4.9 | 6 | 40 |
| 37 | 5.1 | 18 | 5.1 | 8 | 40 |
| 40 | 5.1 | 18 | 5.1 | 6 | 40 |
| 18 | 4.9 | 20 | 4.9 | 4 | 50 |
| 27 | 4.9 | 16 | 5.3 | 4 | 50 |
| *Average value: Run 11 had load condition: pH 5.3, cond 4 mS/cm @ 50 g/L loading Run 24 had load condition: pH 4.9, cond 4 mS/cm @ 30 g/L loading | | | | | |

LC-MS results indicated no lipases present in the CEX pool of all the samples tested based on a database search of known peptide sequences. The detection limit of LC-MS in 1 mg DS was assessed by spiking in 48 different human proteins (6 to 83 kDa) ranging from 500 amoles to 50 pmoles. At least two unique peptides were identified for as low as 0.6 ppm spiked-in protein (data not shown).

Five lipases including phospholipase B-like 2 (PLBL2), lipoprotein lipase (LPL), phospholipase A2 XV (LPLA2), phospholipase A2 VII (LP-PLA2), and lysosomal acid lipase A (LAL/LIPA) were identified in each CEX strip sample and relatively quantified (see **Table 8**). These results suggest strong binding of lipases to the CEX resin in the elution buffer range tested (pH 4.9 - 5.4, conductivity 15 - 21 mS/cm, which corresponds to sodium chloride concentration of 135 - 195 mM).

**Table 8. Relative quantification of endogenous lipases in mAb4 CEX Strips**

| Run # | PLBL2 (ppm) | LPL (ppm) | LPLA2 (ppm) | LP-PLA2 (ppm) | LAL (ppm) |
|-------|-------------|-----------|-------------|---------------|-----------|
| 10&13 | 41.39 | 55.22 | 1.39 | 1.82 | 82.86 |
| 19&20 | 19.37 | 27.44 | 0.68 | 0.88 | 41.66 |
| 11&24 | 113.20 | 171.50 | 4.20 | 6.99 | 260.38 |
| 12 | 60.74 | 89.43 | 2.09 | 2.45 | 107.26 |
| 25 | 80.92 | 114.81 | 2.87 | 4.10 | 192.71 |
| 1&2 | 17.69 | 24.00 | 0.52 | 0.74 | 33.04 |
| 34 | 29.76 | 39.83 | 1.09 | 1.65 | 65.75 |
| 37 | 37.18 | 47.43 | 1.19 | 1.69 | 81.12 |
| 40 | 31.52 | 41.89 | 1.04 | 1.47 | 64.53 |
| 18 | 18.99 | 22.13 | 0.68 | 1.04 | 39.29 |
| 27 | 2.00 | 5.85 | 0.12 | 0.07 | 9.25 |

**Example 6: PS-80 stability increased as host cell lipases were removed**

PS-80 stability was assessed by measuring the PS-80 concentration of a PS80-containing solution over a specified time at specified temperatures. A significant change in PS-80 concentration is defined as two consecutive results outside of a $\pm$ 0.02 mg/mL PS-80 concentration range (*i.e.*, assay variability) compared to the time zero result.

The mAb4 drug substance (DS) is a PS-80-containing solution made via a formulation step, which entails the separate additions of the 49% (w/w) sucrose and 85mM methionine stock solution, and 10% (w/w) PS-80 stock solution to achieve a final DS concentration of 50 mg/mL mAb4 in 10 mM Histidine buffer (pH 5.5), 10 mM Methionine, 7% (w/v) sucrose, and 0.02% (w/v) PS-80.

The PS-80 stability was compared between two mAb4 DS samples that were generated from a two-column and a three-column purification scheme. The two-column purification scheme included Protein A and AEX. The resulting AEX pool (AEXP) was formulated into DS and is referred to as "AEXP DS." The three-column purification scheme included Protein A, AEX, and CEX. The resulting CEX pool (CEXP) was formulated into DS and is referred to as "CEXP DS." Furthermore, a placebo containing the same DS formulation without protein was used as a negative control throughout the study.

Placebo, AEXP DS, and CEXP DS were filled into separate glass vials at a 2.2 ml fill volume and capped with a rubber stopper to simulate the storage of the drug product. Vials were placed in the following stability chambers:

- 5°C $\pm$ 3°C
- 25°C $\pm$ 3°C, 60% $\pm$ 5% relative humidity (RH)
- 40°C $\pm$ 2°C, 75% $\pm$ 5% relative humidity (RH)

Samples were pulled and tested for PS-80 concentration at 2-week intervals up to 12 weeks and 6 months (26 weeks).

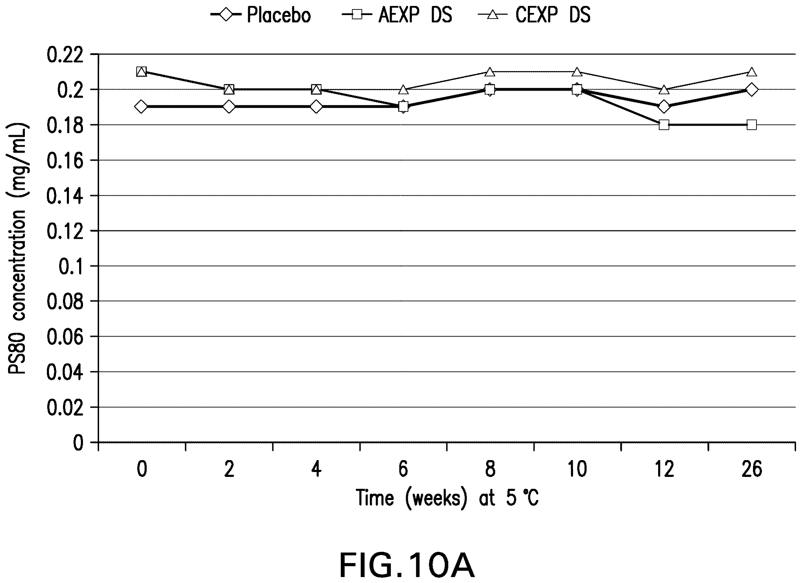

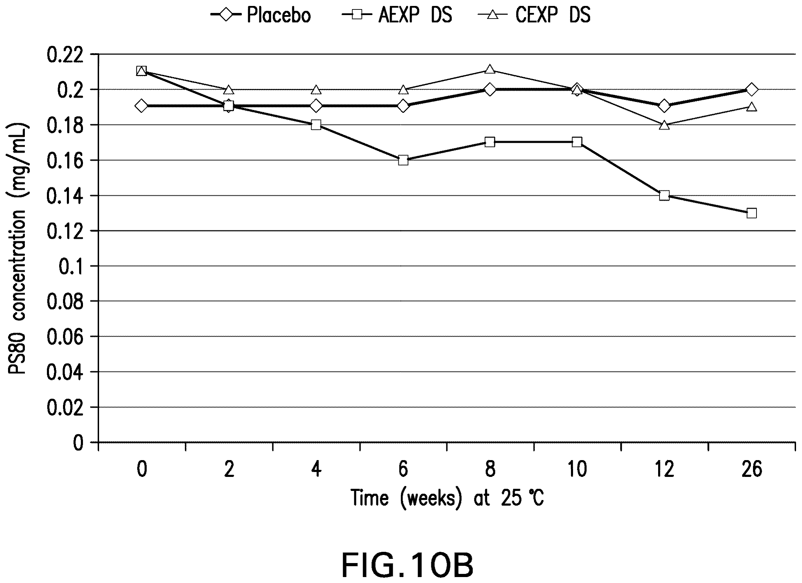

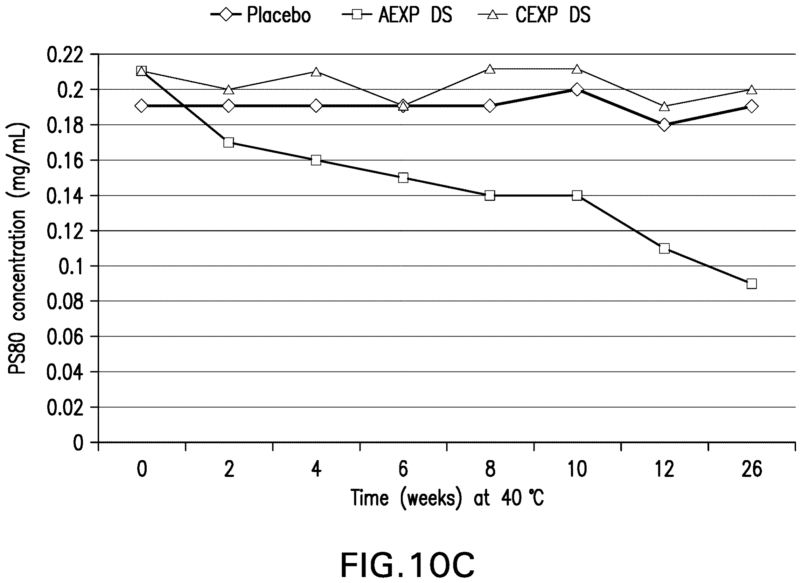

As shown in **FIG. 10A,** the PS-80 concentration in AEXP DS decreased from 0.21 (0 week) to 0.18 mg/mL (12 weeks) at 5°C. The degradation of PS-80 increased as the storage temperature increased. For example, at 25°C, the PS-80 concentration in AEXP DS decreased from 0.21 (0 week) to 0.18 mg/mL (4 weeks) and 0.15 mg/mL (26 weeks) (**FIG. 10B**); at 40°C, the PS-80 concentration in AEXP DS decreased from 0.21 (0 week) to 0.17 mg/mL (2 weeks) and 0.09 mg/mL (26 weeks) (**FIG. 10C**). On the other hand, the PS-80 concentration in CEXP DS did not change significantly over time under all three different temperatures, which is comparable to Placebo.

In parallel with this PS-80 stability study, the in-process intermediates of the same batch along with the corresponding chromatography strip samples were tested for lipase identification by liquid chromatography-mass spectrometry (LC-MS) (**Table 9**). PLBL2 and LPL were found in AEXP but absent from CEXP. Both PLBL2 and LPL were present in the AEX strip and CEX strip, suggesting strong binding of the lipases to the resin. Therefore, it is hypothesized that the presence of PLBL2 and LPL in the AEXP could be one potential cause for the PS-80 concentration decline at 5-40°C in the AEXP DS. Adding a third CEX column can effectively remove these lipases and improve the PS-80 stability in the CEXP DS.

**Table 9. Relative quantification of endogenous PLBL2 and LPL in mAb4 process intermediates**

| Sample | PLBL2 (ppm) Peptide: LTFPTGR (SEQ ID NO:1) | LPL (ppm) Peptide: LVAALYK (SEQ ID NO:2) |
|---|---|---|
| HCCF | 734 | 377.4 |
| PAP | 10.7 | 81.9 |
| PA FT 1 | 190.3 | 65 |
| FNVIP | 10.8 | 39.6 |
| AEXP | 1.2 | 4.7 |
| AEX Strip | 2408.3 | 3398.6 |
| CEXP | Not detectable | Not detectable |
| CEX Strip | 135.2 | 538.9 |
| DS | Not detectable | Not detectable |

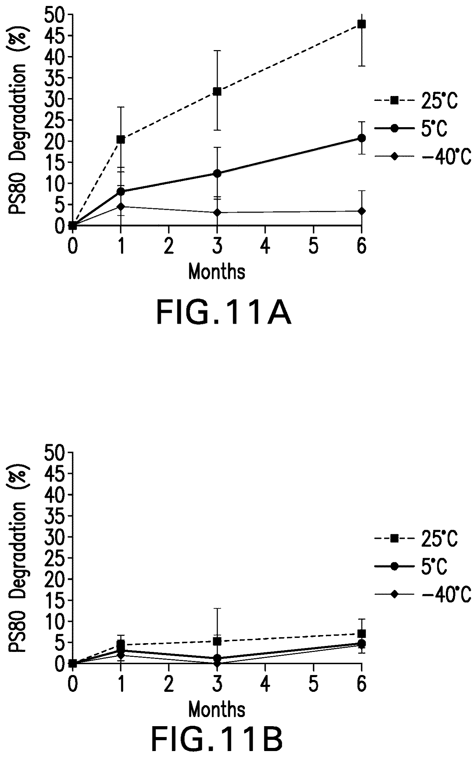

DS from four mAb4 purification batches utilizing only two chromatography steps (Protein A and AEX) were placed on a stability study at -40 °C, 5 °C, and 25 °C for up to six months (**FIG. 11A**). The average PS80 degradation at 25 °C was approximately 20% after just 1 month and approximately 45% after 6 months. DS from three mAb4 purification batches utilizing three chromatography steps (Protein A, AEX, and CEX) were placed on a stability study at -40 °C, 5 °C, and 25 °C for up to six months (**FIG. 11B**). The average PS80 degradation at 25 °C did not go above 10% after six months. These results further support the claim that CEX removes an impurity (such as lipases) and improves PS-80 stability.

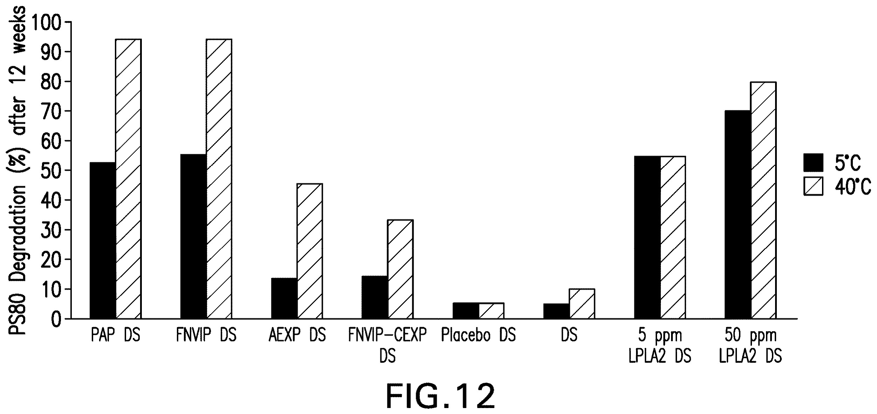

To determine whether the AEX step may be skipped in the whole process, material following the Protein A step, specifically, filtered neutralized viral inactivated pool (FNVIP) was loaded on the CEX column. The resulting CEX pool was formulated into DS and named "FNVIP-CEXP DS." Furthermore, one of the lipases identified in the CEX strip, LPLA2, was spiked into DS at two concentrations, 5 ppm and 50 ppm, as positive lipase controls. These DS samples, along with DS from protein A pool (PAP), FNVIP, AEXP, placebo (the same formulation without DS), and the three-column purification process (Protein A, AEX, and CEX), were placed on a stability study at 5 °C and 40 °C (**FIG. 12**). After 12 weeks at 40 °C, PAP DS, FNVIP DS, and both LPLA2-spiked DS samples experienced over 50% PS80 degradation, while the placebo DS and standard three-column process DS remained at or below 10% PS80 degradation. These results indicate that lipases, such as LPLA2, cause PS80 degradation and are more concentrated in the PAP and FNVIP of the purification process. The AEXP DS and FNVIP-CEXP DS PS80 degraded approximately 30 - 40 % after 12 weeks at 40 °C, suggesting a three-column purification process (Protein A, AEX, and CEX) is necessary to fully remove the residual lipase from mAb4 DS. WHAT IS FEATURED IS:

1. A method of separating a host cell lipase from a production protein through a chromatographic process, comprising:

(a) passing a load fluid comprising the lipase and the production protein through a chromatographic resin under a loading operating condition; and
(b) collecting the production protein in a flowthrough;

wherein separation factor ($\alpha$) is the ratio of the partition coefficient ($K_p$) for the lipase to the $K_p$ for the production protein, and wherein log $\alpha$ is larger than 0.5 under the loading operating condition.

2. The method of feature 1, wherein log $\alpha$ is larger than 1.0 under the loading operating condition.

3. A method of separating a host cell lipase from a production protein through a chromatographic process, comprising:

(a) passing a load fluid comprising the lipase and the production protein through a chromatographic resin; and
(b) eluting the production protein from the chromatographic resin with an elution solution under an elution operating condition;

wherein separation factor ($\alpha$) is the ratio of the partition coefficient ($K_p$) for the lipase to the $K_p$ for the production protein,

and wherein log $\alpha$ is larger than 0.5 under the elution operating condition.

4. The method of feature 3, wherein log $\alpha$ is larger than 1.0 under the elution operating condition.

5. The method of any one of features 1-4, wherein the log $K_p$ for the lipase is larger than 1.0.

6. The method of any one of features 1-4, wherein the log $K_p$ for the lipase is larger than 1.5.

7. The method of any one of features 1-6, wherein the lipase is a CHO cell lipase.

8. The method of any one of features 1-7, wherein the lipase is selected from the group consisting of phospholipase B-like 2 (PLBL2), lipoprotein lipase (LPL), lysosomal phospholipase A2 (LPLA2), phospholipase A2 VII (LP-PLA2), and lysosomal acid lipase A (LAL).

9. The method of any one of features 1-7, wherein the lipase is PLBL2.

10. The method of any one of features 1-7, wherein the lipase is LPL.

11. The method of any one of features 1-7, wherein the lipase is LPLA2.

12. The method of any one of features 1-7, wherein the lipase is LP-PLA2.

13. The method of any one of features 1-7, wherein the lipase is LAL.

14. The method of any one of features 1-13, wherein the chromatographic resin is an ion exchange (IEX) resin.

15. The method of feature 14, wherein the IEX resin is a cation exchange (CEX) resin.

16. The method of feature 15, wherein the CEX resin is a mixed mode CEX resin.

17. The method of feature 15 or 16, wherein the pH of the operating condition is (a) below about 6.0; (b) below about 5.5; (c) below about 5.0; (d) from about 4.5 to about 5.5; (e) from about 4.5 to about 5.0; (f) from about 5.0 to about 5.5; or (g) from about 4.9 to about 5.3.

18. The method of feature 14, wherein the IEX resin is an anion exchange (AEX) resin.

19. The method of feature 18, wherein the AEX resin is a mixed mode AEX resin.

20. The method of feature 18 or 19, wherein the pH of the operating condition is (a) above about 6.5; (b) above about 6.9; (c) above about 7.2; (d) from about 6.9 to about 7.9; (e) from about 7.2 to about 7.5; or (f) from about 7.5 to about 7.8.

21. The method of any one of features 1-13, wherein the chromatographic resin is a hydrophobic interaction (HIC) resin.

22. The method of any one of features 1-21, wherein the operating condition further comprises modulating ion strength and/or conductivity of the operating solution by adding a salt.

23. The method of feature 22, wherein the salt in the operating solution is selected from the group consisting of sodium chloride, sodium acetate, sodium phosphate, ammonium sulfate, sodium sulfate, and Tris-HCl.

24. The method of feature 23, wherein the salt is sodium chloride.

25. The method of feature 24, wherein the concentration of sodium chloride in the operating solution is from about 100 mM to about 225 mM, the chromatographic resin is CEX, the pH of the operating condition is from about 5.0 to about 6.0.

26. The method of feature 24, wherein the concentration of sodium chloride in the operating solution is from about 150 mM to about 180 mM, the chromatographic resin is CEX, the pH of the operating condition is from about 5.0 to about 6.0.

27. The method of feature 23, wherein the salt is sodium acetate.

28. The method of feature 27, wherein the concentration of sodium acetate in the operating solution is from about 100 mM to about 200 mM, the chromatographic resin is AEX, the pH of the operating condition is from about 6.9 to about 7.8.

29. The method of feature 23, wherein the salt is sodium sulfate.

30. The method of feature 29, wherein the concentration of sodium sulfate in the operating solution is from about 500 mM to about 620 mM, the chromatographic resin is HIC, the pH of the operating condition is about 7.

31. The method of feature 29, wherein the concentration of sodium sulfate in the operating solution is from about 510 mM to about 560 mM, the chromatographic resin is HIC, the pH of the operating condition is about 7.

32. A method of separating PLBL2 from a production protein through a mixed mode AEX chromatographic process, comprising:

    (a) passing a load fluid comprising PLBL2 and the production protein through a mixed mode AEX resin; and
    (b) collecting the production protein in a flowthrough;

wherein the pH of the load fluid is from about pH 7.2 to about pH 7.6, and wherein the load fluid does not comprise a salt.

33. A method of separating PLBL2 from a production protein through a CEX chromatographic process, comprising:

    (a) passing a load fluid comprising PLBL2 and the production protein through a CEX resin; and
    (b) eluting the production protein from the CEX resin with an elution solution;

wherein the pH of the elution solution is from about pH 4.9 to about pH 5.3, and wherein the elution solution further comprises from about 120 mM to about 175 mM sodium chloride.

34. The method of feature 33, wherein the pH is about 5.1, and the concentration of sodium chloride is about 150 mM.

35. The method of feature 33, wherein the pH is about 5.1, and the concentration of sodium chloride is about 165 mM.

36. The method of any one of features 1-35, wherein the load fluid is an eluate from a prior chromatographic process.

37. The method of feature 36, wherein the prior chromatographic process comprises an affinity chromatography.

38. The method of feature 36, wherein the prior chromatographic process comprises an affinity chromatography followed by a non-affinity chromatography.

39. The method of feature 37 or 38, wherein the affinity chromatography is a protein A chromatography.

40. The method of feature 38, wherein the non-affinity chromatography is an AEX chromatography.

41. A method of improving polysorbate-80 (PS-80) stability in a production protein formulation, comprising:

    (a) passing a load fluid comprising a host cell lipase and the production protein through a chromatographic resin under a loading operating condition;
    (b) collecting the production protein in a flowthrough; and
    (c) formulating the production protein so that the production protein formulation is a PS-80-containing solution;

wherein separation factor ($\alpha$) is the ratio of the partition coefficient ($K_p$) for the lipase to the $K_p$ for the production protein, and wherein log $\alpha$ is larger than 0.5 under the loading operating condition.

42. The method of feature 41, wherein log $\alpha$ is larger than 1.0 under the loading operating condition.

43. A method of improving polysorbate-80 (PS-80) stability in a production protein formulation, comprising:

(a) passing a load fluid comprising a host cell lipase and the production protein through a chromatographic resin; and
(b) eluting the production protein from the chromatographic resin with an elution solution under an elution operating condition; and
(c) formulating the production protein so that the production protein formulation is a PS-80-containing solution;

wherein separation factor ($\alpha$) is the ratio of the partition coefficient ($K_p$) for the lipase to the $K_p$ for the production protein, and wherein log $\alpha$ is larger than 0.5 under the elution operating condition.

44. The method of feature 43, wherein log $\alpha$ is larger than 1.0 under the elution operating condition.

45. The method of any one of features 41-44, wherein the log $K_p$ for the lipase is larger than 1.0.

46. The method of any one of features 41-44, wherein the log $K_p$ for the lipase is larger than 1.5.

47. The method of any one of features 41-46, wherein the lipase is a CHO cell lipase.

48. The method of any one of features 41-47, wherein the lipase is selected from the group consisting of phospholipase B-like 2 (PLBL2), lipoprotein lipase (LPL), lysosomal phospholipase A2 (LPLA2), phospholipase A2 VII (LP-PLA2), and lysosomal acid lipase A (LAL).

49. The method of any one of features 41-47, wherein the lipase is PLBL2.

50. The method of any one of features 41-47, wherein the lipase is LPL.

51. The method of any one of features 41-47, wherein the lipase is LPLA2.

52. The method of any one of features 41-47, wherein the lipase is LP-PLA2.

53. The method of any one of features 41-47, wherein the lipase is LAL.

54. The method of any one of features 41-53, wherein the chromatographic resin is an IEX resin.

55. The method of feature 54, wherein the IEX resin is a CEX resin.

56. The method of feature 55, wherein the CEX resin is a mixed mode CEX resin.

57. The method of feature 55 or 56, wherein the pH of the operating condition is (a) below about 6.0; (b) below about 5.5; (c) below about 5.0; (d) from about 4.5 to about 5.5; (e) from about 4.5 to about 5.0; (f) from about 5.0 to about 5.5; or (g) from about 4.9 to about 5.3.

58. The method of feature 54, wherein the IEX resin is an AEX resin.

59. The method of feature 58, wherein the AEX resin is a mixed mode AEX resin.

60. The method of feature 58 or 59, wherein the pH of the operating condition is (a) above about 6.5; (b) above about 6.9; (c) above about 7.2; (d) from about 6.9 to about 7.9; (e) from about 7.2 to about 7.5; or (f) from about 7.5 to about 7.8.

61. The method of any one of features 41-53, wherein the chromatographic resin is a HIC resin.

62. The method of any one of features 41-61, wherein the operating condition further comprises modulating ion strength and/or conductivity of the operating solution by adding a salt.

63. The method of feature 62, wherein the salt in the operating solution is selected from the group consisting of sodium chloride, sodium acetate, sodium phosphate, ammonium sulfate, sodium sulfate, and Tris-HCl.

64. The method of feature 63, wherein the salt is sodium chloride.

65. The method of feature 64, wherein the concentration of sodium chloride in the operating solution is from about 100 mM to about 225 mM, the chromatographic resin is CEX, the pH of the operating condition is from about 5.0 to about 6.0.

66. The method of feature 64, wherein the concentration of sodium chloride in the operating solution is from about 150 mM to about 180 mM, the chromatographic resin is CEX, the pH of the operating condition is from about 5.0 to about 6.0.

67. The method of feature 63, wherein the salt is sodium acetate.

68. The method of feature 67, wherein the concentration of sodium acetate in the operating solution is from about 100 mM to about 200 mM, the chromatographic resin is AEX, the pH of the operating condition is from about 6.9 to about 7.8.

69. The method of feature 63, wherein the salt is sodium sulfate.

70. The method of feature 69, wherein the concentration of sodium sulfate in the operating solution is from about 500 mM to about 620 mM, the chromatographic resin is HIC, the pH of the operating condition is about 7.

71. The method of feature 69, wherein the concentration of sodium sulfate in the operating solution is from about 510 mM to about 560 mM, the chromatographic resin is HIC, the pH of the operating condition is about 7.

72. A method of improving polysorbate-80 (PS-80) stability in a production protein formulation, comprising:

    (a) passing a load fluid comprising the production protein through a mixed mode AEX resin;
    (b) collecting the production protein in a flowthrough; and
    (c) formulating the production protein so that the production protein formulation is a PS-80-containing solution;

wherein the pH of the load fluid is from about pH 7.2 to about pH 7.6, and wherein the load fluid does not comprise a salt.

73. A method of improving polysorbate-80 (PS-80) stability in a production protein formulation, comprising:

    (a) passing a load fluid comprising the production protein through a CEX resin;
    (b) eluting the production protein from the CEX resin with an elution solution; and
    (c) formulating the production protein so that the production protein formulation is a PS-80-containing solution;

wherein the pH of the elution solution is from about pH 4.9 to about pH 5.3, and wherein the elution solution further comprises from about 120 mM to about 175 mM sodium chloride.

74. The method of feature 73, wherein the pH is about 5.1, and the concentration of sodium chloride is about 150 mM.

75. The method of feature 73, wherein the pH is about 5.1, and the concentration of sodium chloride is about 165 mM.

76. The method of any one of features 41-75, wherein the load fluid is an eluate from a prior chromatographic process.

77. The method of feature 76, wherein the prior chromatographic process comprises an affinity chromatography.

78. The method of feature 76, wherein the prior chromatographic process comprises an affinity chromatography followed by a non-affinity chromatography.

79. The method of feature 77 or 78, wherein the affinity chromatography is a protein A chromatography.

80. The method of feature 78, wherein the non-affinity chromatography is an AEX chromatography.

81. A method of separating a host cell lipase from a production protein through a CEX chromatographic process, comprising:

(a) passing a load fluid comprising the host cell lipase and the production protein through a CEX resin; and
(b) eluting the production protein from the CEX resin with an elution solution;

wherein the pH of the elution solution is from about pH 4.9 to about pH 5.4, and wherein the conductivity of the elution solution is from about 15 mS/cm to about 21 mS/cm.

82. A method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 4.9 to about pH 5.4, and wherein the conductivity of the elution solution is from about 15 mS/cm to about 21 mS/cm.

83. A method of separating a host cell lipase from a production protein through a CEX chromatographic process, comprising:

(a) passing a load fluid comprising the host cell lipase and the production protein through a CEX resin; and
(b) eluting the production protein from the CEX resin with an elution solution;

wherein the pH of the elution solution is from about pH 4.9 to about pH 5.4, and wherein the elution solution further comprises from about 135 mM to about 195 mM sodium chloride.

84. A method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 4.9 to about pH 5.4, and wherein the elution solution further comprises from about 135 mM to about 195 mM sodium chloride.

85. A method of separating LPLA2 from a production protein through a CEX chromatographic process, comprising:

(a) passing a load fluid comprising LPLA2 and the production protein through a CEX resin; and
(b) eluting the production protein from the CEX resin with an elution solution;

wherein the pH of the elution solution is from about pH 5.0 to about pH 5.4, and wherein the elution solution further comprises from about 150 mM to about 275 mM sodium chloride.

86. The method of feature 85, wherein the pH is about 5.1, and the concentration of sodium chloride is about 150 mM.

87. The method of feature 85, wherein the pH is about 5.1, and the concentration of sodium chloride is about 200 mM.

88. The method of feature 85, wherein the pH is about 5.1, and the concentration of sodium chloride is about 250 mM.

89. A method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 5.0 to about pH 5.4, and wherein the elution solution further comprises from about 150 mM to about 275 mM sodium chloride.

90. The method of feature 89, wherein the pH is about 5.1, and the concentration of sodium chloride is about 200 mM.

91. The method of feature 89, wherein the pH is about 5.1, and the concentration of sodium chloride is about 250 mM.

92. The method of any one of features 1-91, wherein the production protein is a therapeutic protein.

93. The method of feature 92, wherein the therapeutic protein is a monoclonal antibody.

94. A pharmaceutical composition comprising a therapeutic protein and less than 1 ppm of a host cell lipase.

95. The pharmaceutical composition of feature 94, comprising less than 0.1, 0.2, 0.3, 0.4, 0.5, 0.6, 0.7, 0.8, or 0.9 ppm of a host cell lipase.

96. The pharmaceutical composition of feature 94 or 95, wherein the level of the host cell lipase is measured by liquid chromatography-mass spectrometry (LC-MS).

97. The pharmaceutical composition of any one of features 94-96, wherein the pharmaceutical composition is an eluate from a CEX chromatography process using an elution solution selected from the group consisting of:

(a) an elution solution with a pH from about 4.9 to about 5.3, comprising from about 120 mM to about 175 mM sodium chloride;
(b) an elution solution with a pH of about 5.1, comprising about 150 mM sodium chloride;
(c) an elution solution with a pH of about 5.1, comprising about 165 mM sodium chloride;
(d) an elution solution with a pH from about 4.9 to about 5.4 and a conductivity from about 15 mS/cm to about 21 mS/cm;
(e) an elution solution with a pH from about 4.9 to about 5.4, comprising from about 135 mM to about 195 mM sodium chloride;
(f) an elution solution with a pH from about pH 5.0 to about pH 5.4, comprising from about 150 mM to about 275 mM sodium chloride;
(g) an elution solution with a pH of about 5.1, comprising about 200 mM sodium chloride; and
(h) an elution solution with a pH of about 5.1, comprising about 250 mM sodium chloride.

98. The pharmaceutical composition of feature 97, wherein the CEX chromatography is preceded by an AEX chromatography operated in a flowthrough mode.

99. The pharmaceutical composition of any one of features 94-98, wherein the lipase is selected from the group consisting of PLBL2, LPL, LPLA2, LP-PLA2, and LAL.

100. The pharmaceutical composition of any one of features 94-99, wherein the therapeutic protein is a monoclonal antibody.

**Claims**

1. A method of improving polysorbate-80 (PS-80) stability in a production protein formulation, comprising:

(1)

(a) passing a load fluid comprising the lipase and the production protein through a chromatographic resin under a loading operating condition;
(b) collecting the production protein in a flowthrough; and
(c) formulating the production protein so that the production protein formulation is a PS-80-containing solution;
wherein separation factor ($\alpha$) is the ratio of the partition coefficient ($K_p$) for the lipase to the $K_p$ for the production protein, and wherein log $\alpha$ is (i) larger than 0.5 or (ii) larger than 1.0 under the loading operating condition;

wherein optionally the log $K_p$ for the lipase is (i) larger than 1.0 or (ii) larger than 1.5;

wherein optionally the lipase is a CHO cell lipase selected from the group consisting of phospholipase B-like 2 (PLBL2), lipoprotein lipase (LPL), lysosomal phospholipase A2 (LPLA2), phospholipase A2 VII (LP-PLA2), and lysosomal acid lipase A (LAL);

or

(2)

(a) passing a load fluid comprising the lipase and the production protein through a chromatographic resin;

(b) eluting the production protein from the chromatographic resin with an elution solution under an elution operating condition; and

(c) formulating the production protein so that the production protein formulation is a PS-80-containing solution;

wherein separation factor ($\alpha$) is the ratio of the partition coefficient ($K_p$) for the lipase to the $K_p$ for the production protein, and wherein log $\alpha$ is (i) larger than 0.5 or (ii) larger than 1.0 under the elution operating condition;

wherein optionally the log $K_p$ for the lipase is (i) larger than 1.0 or (ii) larger than 1.5;

wherein optionally the lipase is a CHO cell lipase selected from the group consisting of phospholipase B-like 2 (PLBL2), lipoprotein lipase (LPL), lysosomal phospholipase A2 (LPLA2), phospholipase A2 VII (LP-PLA2), and lysosomal acid lipase A (LAL).

2. The method of claim 1, wherein the chromatographic resin is

(1) an ion exchange (IEX) resin; wherein optionally the IEX resin is

(a) a cation exchange (CEX) resin; wherein optionally the CEX resin is a mixed mode CEX resin; wherein optionally the pH of the operating condition is (i) below about 6.0; (ii) below about 5.5; (iii) below about 5.0; (iv) from about 4.5 to about 5.5; (v) from about 4.5 to about 5.0; (vi) from about 5.0 to about 5.5; or (vii) from about 4.9 to about 5.3; or

(b) an anion exchange (AEX) resin; wherein optionally the AEX resin is a mixed mode AEX resin; wherein optionally the pH of the operating condition is (i) above about 6.5; (ii) above about 6.9; (iii) above about 7.2; (iv) from about 6.9 to about 7.9; (v) from about 7.2 to about 7.5; or (vi) from about 7.5 to about 7.8; or

(2) a hydrophobic interaction (HIC) resin.

3. The method of claim 1 or 2, wherein the operating condition further comprises modulating ion strength and/or conductivity of the operating solution by adding a salt; wherein optionally the salt in the operating solution is selected from the group consisting of sodium chloride, sodium acetate, sodium phosphate, ammonium sulfate, sodium sulfate, and Tris-HCl.

4. The method of claim 3, wherein

(1) the salt is sodium chloride, the concentration of sodium chloride in the operating solution is from about 100 mM to about 225 mM, the chromatographic resin is CEX, the pH of the operating condition is from about 5.0 to about 6.0;

(2) the salt is sodium chloride, the concentration of sodium chloride in the operating solution is from about 150 mM to about 180 mM, the chromatographic resin is CEX, the pH of the operating condition is from about 5.0 to about 6.0;

(3) the salt is sodium acetate, the concentration of sodium acetate in the operating solution is from about 100 mM to about 200 mM, the chromatographic resin is AEX, the pH of the operating condition is from about 6.9 to about 7.8;

(4) the salt is sodium sulfate, the concentration of sodium sulfate in the operating solution is from about 500 mM to about 620 mM, the chromatographic resin is HIC, the pH of the operating condition is about 7; or

(5) the salt is sodium sulfate, the concentration of sodium sulfate in the operating solution is from about 510 mM to about 560 mM, the chromatographic resin is HIC, the pH of the operating condition is about 7.

5. A method of improving polysorbate-80 (PS-80) stability in a production protein formulation, comprising:

(a) passing a load fluid comprising PLBL2 and the production protein through a mixed mode AEX resin;
(b) collecting the production protein in a flowthrough; and
(c) formulating the production protein so that the production protein formulation is a PS-80-containing solution;

wherein the pH of the load fluid is from about pH 7.2 to about pH 7.6, and wherein the load fluid does not comprise a salt.

6. A method of improving polysorbate-80 (PS-80) stability in a production protein formulation, comprising:

(a) passing a load fluid comprising PLBL2 and the production protein through a CEX resin;
(b) eluting the production protein from the CEX resin with an elution solution; and
(c) formulating the production protein so that the production protein formulation is a PS-80-containing solution;

wherein:

(i) the pH of the elution solution is from about pH 4.9 to about pH 5.3, and the elution solution further comprises from about 120 mM to about 175 mM sodium chloride; optionally wherein the pH is about 5.1 and the concentration of sodium chloride is (i) about 150 mM or (ii) about 165 mM; or
(ii) the pH of the elution solution is from about pH 4.9 to about pH 5.4; and the conductivity of the elution solution is from about 15 mS/cm to about 21 mS/cm; or wherein the elution solution comprises from about 135 mM to about 195 mM sodium chloride.

7. The method of claim 6, wherein the load fluid is an eluate from a prior chromatographic process; wherein optionally the prior chromatographic process comprises (i) an affinity chromatography; wherein optionally the affinity chromatography is a protein A chromatography; or (ii) an affinity chromatography followed by a non-affinity chromatography; wherein optionally the affinity chromatography is a protein A chromatography and the non-affinity chromatography is an AEX chromatography.

8. A method of separating LPLA2 from a production protein through a CEX chromatographic process, comprising:

(a) passing a load fluid comprising LPLA2 and the production protein through a CEX resin; and
(b) eluting the production protein from the CEX resin with an elution solution;

wherein the pH of the elution solution is from about pH 5.0 to about pH 5.4, and wherein the elution solution further comprises from about 150 mM to about 275 mM sodium chloride.

9. The method of claim 8, wherein the pH is about 5.1, and the concentration of sodium chloride is about 150 mM.

10. The method of claim 8, wherein the pH is about 5.1, and the concentration of sodium chloride is about 200 mM.

11. The method of claim 8, wherein the pH is about 5.1, and the concentration of sodium chloride is about 250 mM.

12. A method of improving PS-80 stability in a production protein formulation, comprising:

(a) passing a load fluid comprising the production protein through a CEX resin;
(b) eluting the production protein from the CEX resin with an elution solution; and
(c) formulating the production protein so that the production protein formulation is a PS-80-containing formulation;

wherein the pH of the elution solution is from about pH 5.0 to about pH 5.4, and wherein the elution solution further comprises from about 150 mM to about 275 mM sodium chloride.

13. The method of claim 12, wherein the pH is about 5.1, and the concentration of sodium chloride is about 200 mM.

14. The method of claim 12, wherein the pH is about 5.1, and the concentration of sodium chloride is about 250 mM.

FIG.1A

FIG.1B

FIG.1C

FIG.1D

FIG.1E

FIG.1F

FIG.2A

FIG.2B

FIG.2C

FIG.3

FIG.4A

FIG.4B

FIG.5

FIG.6A

FIG.6B

FIG.6C

FIG.7

FIG.8A

FIG.8B

| Source | LogWorth | | PValue |
|---|---|---|---|
| Rec E buff pH(4.9,5.3) | 4.831 | | 0.00001 |
| Rec E buff pH*Rec E buff cond (mS/cm) | 3.095 | | 0.00080 |
| Rec E buff cond (mS/cm)(16,20) | 2.271 | | 0.00536 |
| Rec E buff cond (mS/cm)*Rec E buff cond (mS/cm) | 2.082 | | 0.00827 |
| Rec E buff pH*Rec E buff pH | 1.755 | | 0.01758 |
| Rec E buff pH*Resin load (g/L) | 1.035 | | 0.09231 |
| Resin load (g/L) (30,50) | 0.754 | | 0.17633 |

## FIG.9

FIG.10A

FIG.1OB

FIG.10C

FIG.11A

FIG.11B

FIG.12

EP 4 717 339 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62703097 A
- US 4816567 A
- US 7332582 B
- US 9683048 B
- US 8735553 B
- US 20170088613 A

**Non-patent literature cited in the description**

- **KOHLER et al.** *Nature*, 1975, vol. 256, 495
- **CLACKSON et al.** *Nature*, 1991, vol. 352, 624-628
- **MARKS et al.** *J. Mol. Biol.*, 1991, vol. 222, 581-597
- **PRESTA**. *J. Allergy Clin. Immunol.*, 2005, vol. 116, 731
- **WELSH et al.** *Biotechnol Prog.*, 2014, vol. 30 (3), 626-635
- **PETROFF et al.** *Biotech Bioeng.*, 2015, vol. 113 (6), 1273-1283